# FASCICULE DE BREVET EUROPEEN

(11) **EP 1 539 734 B1**
(45) Date de publication et mention de la délivrance du brevet: **08.03.2006**
(21) Numéro de dépôt: 03769606.9
(22) Date de dépôt: 10.09.2003
(51) Int. Cl.: C07D 401/06, A61K 31/4709, A61P 31/04, C07D 409/14, C07D 211/60

(54) **DERIVES DE LA QUINOLYL PROPYL PIPERIDINE ET LEUR UTILISATION EN TANT QU'AGENTS ANTIMICROBIENS**
CHINOLYL-PROPYLPIPERIDINDERIVATE UND DEREN VERWENDUNG ALS ANTIMIKROBIELLE WIRKSTOFFE
PIPERIDINE QUINOLYL PROPYL DERIVATIVES AND USE THEREOF AS ANTIMICROBIAL AGENTS

(30) Priorité: 11.09.2002 FR 0211213
(43) Date de publication de la demande: 15.06.2005
(73) Titulaire: NOVEXEL, 93230 Romainville (FR)
(72) Inventeur: BACQUE, Eric, F-91190 Gif sur Yvette (FR); BIGOT, Antony, F-91300 Massy (FR); EL AHMAD, Youssef, F-94000 Creteil (FR); MALLERON, Jean-Luc, F-91460 Marcoussis (FR); MIGNANI, Serge, F-92290 Châtenay-Malabry (FR); RONAN, Baptiste, F-92140 Clamart (FR); TABART, Michel, F-91290 La Norville (FR); VIVIANI, Fabrice, F-95380 Louvres (FR)
(74) Mandataire: Hirsch & Associés
(86) Numéro de dépôt international: PCT/FR2003/002687
(87) Numéro de publication internationale: WO 2004/024713

(56) Documents cités:
- WO-A-00/43383
- WO-A-01/25227
- WO-A-02/40474
- WO-A-02/072572

## Description

La présente invention concerne des dérivés de quinolyl propyl pipéridine de formule générale : 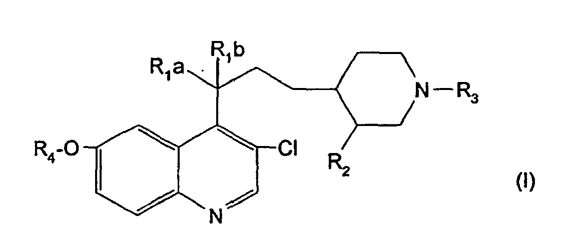
qui sont actifs comme antimicrobiens. L'invention concerne également leur procédé et intermédiaires de préparation et les compositions pharmaceutiques les contenant.

Dans les demandes de brevet WO 99/37635 et WO 00/43383 ont été décrits des dérivés de quinolyl propyl pipéridine antimicrobiens, de formule générale : 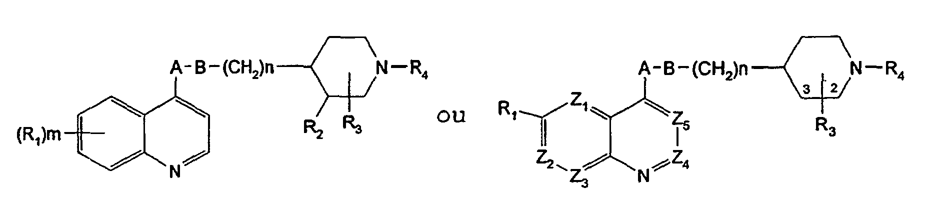
dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, carboxy, alcoyloxycarbonyle, alcoylcarbonyle, alcènyloxycarbonyle, alcènylcarbonyle, hydroxy éventuellement substitué par alcoyle ..., R₄ est un groupe -CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, NR₁₁, CR₆R₇ pour lequel R₆ et R₇ représentent H, thiol, alcoylthio, halo, trifluorométhyle, alcènyle, alcènylcarbonyle, hydroxy, amino, et Z₁ à Z₅ sont N ou CR₁ₐ ...

Par ailleurs, les demandes de brevets WO-A-0125227 et WO-A-0240474 décrivent respectivement des dérivés de type quinolyl propyl pipéridine 3-carboxy ou 3-hydroxy méthyle mais ne comportent aucune substitution en position 3 de la quinoléine et des dérivés de type quinolyl propyl pipéridine 4-carboxy ou 4-hydroxyméthyle comportant diverses possibilités de substitution en position 3 de la quinoléine.

Enfin, dans la demande de brevet européen EP30044 ont été décrits des dérivés de quinoléine utiles comme cardiovasculaires, répondant à la formule générale : 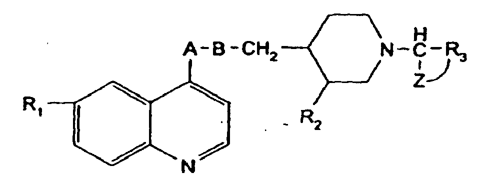
dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé, et c'est ce qui fait l'objet de la présente invention, que les produits de formule générale (I) pour lesquels :
R₁ₐ est un atome d'hydrogène ou d'halogène ou un radical hydroxy, amino, alcoylamino, dialcoylamino, hydroxyamino, alcoyloxyamino ou alcoyl alcoyloxy amino et R_{1b} est un atome d'hydrogène, ou R₁ₐ et R_{1b} forment un groupement oxo,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, par un radical cycloalcoylthio dont la partie cyclique contient 3 à 7 chaînons, pouvant lui-même porter un ou plusieurs substituants choisis parmi halogène et trifluorométhyle, ou par un radical hétéroarylthio de 5 à 6 chaînons comprenant 1 à 4 hétéroatomes choisis parmi l'azote, l'oxygène et le soufre, pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino ou R₃ représente un radical propargyle substitué par un radical phényle pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, ou substitué par un radical cycloalcoyle contenant 3 à 7 chaînons pouvant lui-même porter un ou plusieurs substituants choisis parmi halogène et trifluorométhyle, ou substitué par un radical hétéroaryle de 5 à 6 chaînons comprenant 1 à 4 hétéroatomes choisis parmi l'azote, l'oxygène et le soufre et pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino, et
R₄ représente un radical alcoyle contenant 1 à 6 atomes de carbone, alcényl-CH₂- ou alcynyl-CH₂- dont les parties alcényle ou alcynyle contiennent 2 à 6 atomes de carbone, cycloalcoyle ou cycloalcoyl alcoyle dont la partie cycloalcoyle contient 3 à 8 atomes de carbone,

sous leurs formes isomères, éniantomères et diastéréoisomères, séparées ou en mélanges, ainsi que leurs sels, sont de puissants agents antibactériens.

Il est entendu que les radicaux et portions alcoyle sont en chaîne droite ou ramifiée et contiennent, sauf mention spéciale, 1 à 4 atomes de carbone, et que dans l'alternative où R₁ représente un atome d'halogène ou lorsque R₃ porte un substituant halogène, celui-ci peut être choisi parmi fluor, chlore, brome et iode, fluor étant préféré.

Dans la formule générale ci-dessus, lorsque R₃ porte un substituant hétéroaryle, ce dernier peut être choisi, à titre non limitatif, parmi thiényle, furyle, pyrrolyle, imidazolyle, thiazolyle, oxazolyle, thiadiazolyle, oxadiazolyle, tétrazolyle, pyridyle, pyridazinyle, pyrazinyle et pyrimidinyle.

L'invention a notamment pour objet les dérivés de formule générale (I) telle que définie précédemment, dans laquelle R1a est un radical hydroxy et R₂ₐ est un atome d'hydrogène, ceux dans laquelle R₁ₐ et R₂ₐ forment un groupement oxo, ceux dans laquelle R₄ représente un radical alcoyle comportant de 1 à 6 atomes de carbone, notamment méthyle, ceux dans laquelle R₂ représente un radical carboxy et ceux dans laquelle R₃ représente un radical alcoyle, notamment éthyle, substitué par un radical phénylthio, cycloalcoylthio ou hétéroarylthio éventuellement substitués tels que définis plus haut, plus particulièrement ceux dans laquelle R₃ représente un radical éthyle substitué par un radical thiénylthio, phénylthio substitué par halogène, notamment fluor, ou par trifluorométhyle, cyclohexylthio ou cyclopentylthio.

L'invention a plus particulièrement pour objet les dérivés de formule générale (I) dont les noms suivent :
L'acide -4-[3-hydroxy-3-(3-chloro-6-méthoxyquinolin-4-yl)-propyl]-1-[2-(2,5-difluoro-phénylsulfanyl)-éthyl]-pipéridine-3-carboxylique ;
L'acide -4-[3-hydroxy-3-(3-chloro-6-méthoxyquinolin-4-yl)-propyl]-1-[2-(2-thiénylsulfanyl)-éthyl]-pipéridine=3 carboxylique ;

sous leurs différentes formes isomères, séparées ou en mélanges, ainsi que leurs sels.

Selon l'invention, les produits de formule générale (I) peuvent être obtenus par condensation de la chaîne R₃ sur le dérivé de quinolyl propyl pipéridine de formule générale : 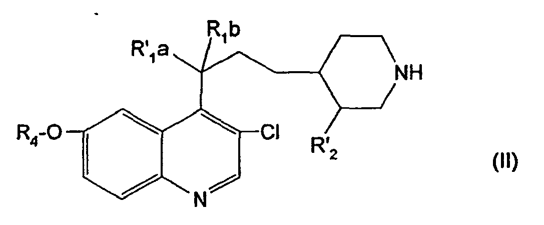
dans laquelle R₄ est défini comme précédemment, soit R'₁ₐ représente un atome d'hydrogène ou un radical hydroxy et R_{1b} représente un atome d'hydrogène soit R'₁ₐ et R_{1b} forment un groupement oxo et R'₂ représente un radical carboxy ou carboxyméthyle protégé, pour obtenir un dérivé de quinolyl propyl pipéridine de formule générale : 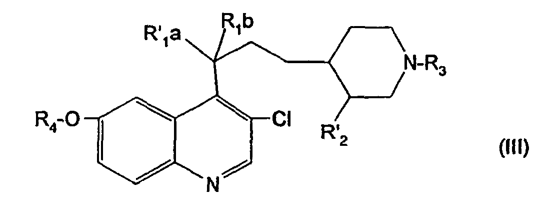
pour lequel R'₁ₐ, R_{1b}, R'₂ et R₄ sont définis comme ci-dessus et R₃ est défini comme précédemment,
puis, le cas échéant, halogénation du dérivé pour lequel R'₁ₐ est un radical hydroxy et R_{1b} est un atome d'hydrogène, si l'on veut obtenir un dérivé pour lequel R'₁ₐ est un atome d'halogène,
ou bien, le cas échéant, transformation du radical hydroxy représenté par R'₁ₐ en un radical oxo, puis, le cas échéant, transformation de celui-ci en un radical hydroxyimino ou alcoyloxyimino, pour obtenir un dérivé de quinolyl propyl pipéridine de formule générale : 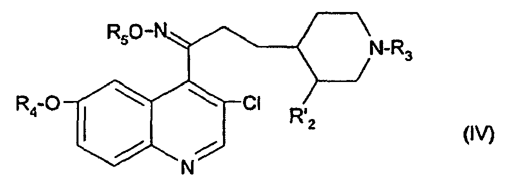
pour lequel R'₂, R₃ et R₄ sont définis comme précédemment, et R₅ est un atome d'hydrogène ou un radical alcoyle, et réduction du dérivé de formule générale (IV) pour lequel R₅ est un atome d'hydrogène en amine, et éventuellement transformation en une amine monoalcoylée ou dialcoylée, ou éventuellement réduction du dérivé de formule générale (IV) pour lequel R₅ est un atome d'hydrogène en hydroxylamine, ou du dérivé de formule générale (IV) pour lequel R₅ est un radical alcoyle en alcoyloxyamine, puis, le cas échéant, pour obtenir le dérivé pour lequel R₁ₐ est alcoyl alcoyloxy amino, transformation du dérivé obtenu pour lequel R₁ₐ est alcoyloxyamino par alcoylation,
puis transformation de R'₂ en un radical carboxy ou carboxyméthyl, et/ou, le cas échéant, réduction du radical carboxy ainsi obtenu ou du radical carboxy protégé que peut représenter R'₂ en un radical hydroxyméthyle et le cas échéant transformation de celui-ci en un radical carboxyméthyle selon les méthodes habituelles,
puis, le cas échéant, séparation des isomères, le cas échéant élimination du radical protecteur d'acide, et/ou, le cas échéant, transformation du produit obtenu en un sel.

La condensation de la chaîne R₃ sur la pipéridine s'effectue avantageusement par action d'un dérivé de formule générale :

R₃-X (V)

dans laquelle R₃ est défini comme précédemment et X représente un atome d'halogène, un radical méthylsulfonyloxy, un radical trifluorométhylsulfonyloxy ou p.toluènesulfonyloxy, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant. De préférence, on fait agir un dérivé pour lequel X est un atome de brome ou d'iode.

Des dérivés de formule (V) sont décrits ou peuvent être préparés comme décrit, par exemple, dans les demandes WO 200125227 ou 200240474.

Lorsque R₃ représente propargyle substitué par phényle, cycloalcoyle ou hétéroaryle, il peut être aussi préférable de condenser un halogénure de propargyle, puis de substituer la chaîne par un radical phényle, cycloalcoyle ou hétéroaryle. Dans cette alternative, la condensation de la chaîne propargylique s'effectue au moyen de bromure de propargyle, dans les conditions énoncées ci-dessus, le cas échéant en présence d'un iodure de métal alcalin comme par exemple l'iodure de potassium ou de sodium.
Lorsqu'il s'agit de la substitution par un radical phényle ou hétéroaryle, la réaction s'effectue par action d'un halogénure dérivé du radical cyclique à substituer, en présence de triéthylamine, en milieu anhydre, éventuellement sans solvant ou dans un solvant tel qu'un amide (diméthylformamide par exemple) ou un nitrile (acétonitrile par exemple) et en présence d'un sel de palladium comme par exemple le tétrakis triphénylphosphine palladium et d'iodure cuivreux, à une température comprise entre 20°C et la température de reflux du solvant.
Lorsqu'il s'agit de la substitution par un groupement cycloalkyle, la réaction s'effectue par action d'un organolithien comme le n-butyllithium ou le tert-butyllithium sur le dérivé propargylique obtenu précédemment, en milieu anhydre dans un éther comme par exemple le tétrahydrofurane à une température comprise entre -78 et 0°C, puis action d'une cycloalcanone suivi de la désoxygénation de l'alcool intermédiaire selon les méthodes classiques.

Il est entendu que, lorsque les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le radical carboxy ou carboxyméthyle protégé représenté par R'₂ peut être choisi parmi les esters facilement hydrolysables. A titre d'exemple peuvent être cités les esters méthyliques, benzyliques, tertiobutyliques, ou bien les esters d'allyle ou de phénylpropyle. Eventuellement la protection du radical carboxy s'effectue simultanément à la réaction. Dans ce cas le produit de formule générale (II) mis en oeuvre porte un radical R'₂ = carboxy ou carboxyméthyle.

L'halogénation conduisant à un dérivé pour lequel R₁ₐ est un atome d'halogène peut être mise en oeuvre en présence d'un trifluorure d'aminosoufre (trifluorure de diéthylamino soufre, trifluorure de bis(2-méthoxyéthyl)amino soufre (Deoxofluor®), trifluorure de morpholino soufre par exemple) ou alternativement en présence de tétrafluorure de soufre. La réaction de fluoration peut être également mise en oeuvre par action d'un agent de fluoration comme un fluorure de soufre [par exemple trifluorure de morpholino soufre, tétrafluorure de soufre (J. Org. Chem., 40, 3808 (1975)), trifluorure de diéthylamino soufre (Tetrahedron, 44, 2875 (1988)), trifluorure de bis(2-méthoxyéthyl)amino soufre (Deoxofluor®). Alternativement la réaction de fluoration peut aussi s'effectuer au moyen d'un agent de fluoration comme l'hexafluoropropyl diéthylamine (JP 2 039 546) ou la N-(chloro-2 trifluoro-1,1,2 éthyl) diéthylamine. La réaction d'halogénation peut également s'effectuer au moyen d'un réactif comme un halogénure de tétra alkylammonium, de tri alkyl benzylammonium ou de tri alkyl phénylammonium ou au moyen d'un halogénure de métal alcalin additionné éventuellement d'un éther couronne.
Lorsque l'on met en oeuvre un halogénure de tétra alkylammonium, ce dernier peut être choisi, à titre d'exemple, parmi les halogénures de tétra méthylammonium, de tétra éthylammonium, de tétra propylammonium, de tétra butylammonium (tétra n-butylammonium par exemple), de tétra pentylammonium, de tétra cyclohexylammonium, de tri éthyl méthylammonium de tri butyl méthylammonium, ou de tri méthyl propylammonium.
On opère dans un solvant organique tel qu'un solvant chloré (par exemple dichlorométhane, dichloréthane, chloroforme) ou dans un éther (tétrahydrofurane, dioxanne par exemple) à une température comprise entre -78 et 40°C (de préférence entre 0 et 30°C). Il est avantageux d'opérer en milieu inerte (argon ou azote notamment).
Il est également possible d'opérer par action d'un agent d'halogénation comme le chlorure de thionyle ou le trichlorure de phosphore dans un solvant organique tel qu'un solvant chloré (dichlorométhane, chloroforme par exemple), à une température comprise entre 0°C et la température de reflux du mélange réactionnel.

La transformation du radical hydroxy en un radical oxo, s'effectue par les méthodes d'oxydation classiques décrites dans la littérature, par exemple par oxydation de D. Swern, J.O.C., 44, 41-48 (1979) notamment en présence de chlorure d'oxalyle et de diméthylsulfoxyde, éventuellement dans un solvant, par exemple le dichlorométhane, à une température comprise entre -60 et 20°C.

La transformation du radical oxo en un radical hydroxyimino ou alcoyloxyimino s'effectue par action d'hydroxylamine ou d'alcoyloxyamine, éventuellement sous forme de chlorhydrate, dans un solvant tel que la pyridine ou un alcool (tel que le méthanol ou l'éthanol) et en présence d'une base azotée telle que la triéthylamine ou la pyridine, à une température comprise entre 0 et 60°C.

La réduction du dérivé de formule générale (IV) pour lequel R₅ est hydrogène en amine s'effectue selon les méthodes habituelles qui n'altèrent pas le reste de la molécule, notamment par action d'un agent réducteur comme par exemple un hydrure (borohydrure alcalin : borohydrure de sodium ou de potassium par exemple ou hydrure d'aluminium et de lithium) en présence ou non d'oxyde de molybdène, en opérant de préférence sous atmosphère inerte (azote ou argon par exemple), dans un solvant organique comme un alcool (méthanol, éthanol, isopropanol par exemple) ou un solvant chloré (par exemple dichorométhane) à une température comprise entre -10 et 40°C.

La réduction du dérivé de formule générale (IV) en hydroxylamine ou en alcoyloxyamine s'effectue notamment en présence d'un acide organique (acide carboxylique comme par exemple l'acide acétique), par action d'un agent réducteur comme par exemple un hydrure choisi parmi le triacétoxyborohydrure de sodium (éventuellement préparé in situ) et le cyanoborohydrure de sodium, de préférence sous atmosphère inerte (azote ou argon par exemple), dans un solvant organique comme un alcool (méthanol, éthanol, isopropanol par exemple) ou un solvant chloré (par exemple dichorométhane) à une température comprise entre -30 et +40°C.

La transformation du radical amino représenté par R₁ₐ en un radical alcoylamino ou dialcoylamino s'effectue selon les méthodes habituelles, notamment par action d'un halogénure d'alcoyle, éventuellement en milieu basique en présence d'une base azotée comme une trialcoylamine (triéthylamine, diisopropyl éthyl amine ...), la pyridine, ou en présence d'un hydrure de métal alcalin (hydrure de sodium), dans un solvant inerte comme un amide (diméthylformamide par exemple) ou un oxyde (diméthylsulfoxyde par exemple), à une température comprise entre 20°C et la température de reflux du mélange réactionnel.

La transformation du radical alcoyloxyamino représenté par R₁ₐ en un radical alcoyl alcoyloxy amino s'effectue selon la méthode décrite ci-dessus pour l'alcoylation de l'amine.

La transformation de R'₂ en un radical carboxy ou carboxyméthyle, s'effectue selon les méthodes habituelles, notamment par hydrolyse acide ou saponification de l'ester R'₂. On fait notamment agir la soude en milieu hydroorganique, par exemple dans un alcool comme le méthanol ou un éther comme le dioxanne, à une température comprise entre 20°C et la température de reflux du mélange réactionnel. On peut également mettre en oeuvre l'hydrolyse en milieu chlorhydrique aqueux à une température comprise entre 20 et 100°C.

La réduction en un radical hydroxyméthyle d'un dérivé pour lequel R'₂ est carboxy protégé peut être effectuée selon les méthodes habituelles, connues de l'homme du métier, qui n'altèrent pas le reste de la molécule, en particulier par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.

La réduction de l'acide libre peut être effectuée selon des méthodes également connues de l'homme du métier, par exemple par hydrogénation en présence d'un catalyseur à base de rhodium ou de ruthénium, par action de hydroborure de sodium en présence d'acide de Lewis ou d'hydrure d'aluminium et de lithium dans l'éther.

La transformation du radical hydroxyméthyle en position 3 de la pipéridine en un radical carboxyméthyle s'effectue selon les méthodes habituelles qui n'altèrent pas le reste de la molécule, notamment par action d'un agent d'halogénation comme par exemple le chlorure de thionyle ou le trichlorure de phosphore ou le tribromure de phosphore, ou d'un agent de tosylation, puis d'un cyanure alcalin, par exemple (cyanure de potassium ou cyanure de sodium, pour préparer le dérivé cyanométhyle correspondant, suivie de l'hydrolyse du nitrile. Lorsque le radical R₁ est un radical amino, il est préférable de protéger préalablement ce radical selon les méthodes connues et citées ci-avant pour R₃.

L'halogénation peut être effectuée dans un solvant chloré (dichlorométhane ou chloroforme par exemple), à une température comprise entre 0°C et la température de reflux du solvant.

Le dérivé de quinolyl propyl pipéridine de formule générale (II), pour lequel R'₁ₐ est un radical hydroxy et R_{1b} est un atome d'hydrogène peut être préparé par oxydation en milieu basique au départ d'un dérivé correspondant pour lequel R'₁ₐ et R_{1b} sont des atomes d'hydrogène, la fonction amine de la pipéridine est protégée intermédiairement et R'₂ est tel que défini précédemment ou représente un radical carboxy ou carboxyméthyle et le cas échéant,reprotection du radical carboxy ou carboxyméthyle. L'oxydation s'effectue par action de l'oxygène, de préférence au sein d'un solvant inerte tel que le diméthylsulfoxyde en présence de tert-butanol et d'une base telle que le tert-butylate de potassium ou dé sodium à une température comprise entre 0 et 100°C.

Le dérivé de quinolyl propyl pipéridine de formule générale (II) dans laquelle R'₁ₐ et R_{1b} forment un groupement oxo peut être préparé de manière analogue à celle indiquée plus haut, par les méthodes d'oxydation classiques, au départ d'un dérivé de formule générale (II) dans laquelle R'₁ₐ représente un radical hydroxy, en protégeant intermédiairement l'azote de la pipéridine.

Le dérivé de quinolyl propyl pipéridine de formule générale (II) pour lequel R'₂ représente un radical carboxyméthyle protégé, et R'₁ₐ et R_{1b} sont des atomes d'hydrogène, peut être préparé par hydrogénation sélective du dérivé de quinolyl propyl pipéridine de formule générale : 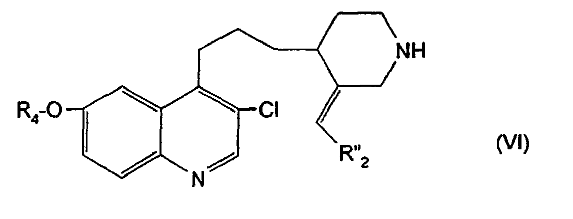
dans laquelle R₄ est défini comme précédemment et R"₂ est le radical carboxy protégé correspondant à R'₂, et dont la fonction amine de la pipéridine est préalablement protégée, sous une pression de 1 à 100 bars et à une température comprise entre 20 et 80°C, dans un solvant comme notamment un alcool (éthanol par exemple) ou un amide (diméthylformamide par exemple) en présence d'un catalyseur, par exemple le palladium sur charbon ou le palladium sur sulfate de baryum. La protection de l'amino de la pipéridine s'effectue selon les méthodes habituelles qui n'altèrent pas le reste de la molécule et compatibles avec la réaction notamment selon les références relatives aux groupement protecteurs citées ci-avant. Le radical protecteur est plus particulièrement le radical benzyloxycarbonyle. Dans ce cas, la réaction d'hydrogénation conduit directement à la déprotection de l'amine.

Le dérivé de quinolyl propyl pipéridine de formule générale (VI) peut être préparé par condensation d'un dérivé de quinoléine de formule générale : 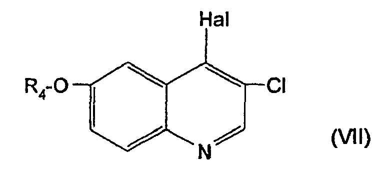
dans laquelle R₄ est défini comme précédemment et Hal représente un atome d'iode ou de brome, sur un dérivé de la pipéridine de formule générale : 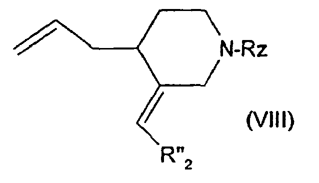
dans laquelle R"₂ est défini comme ci-dessus et R_{z} représente un radical protecteur d'amino.

La réaction s'effectue par action successive d'un organoborane (9-borabicyclo[3,3,1]nonane par exemple) dans un solvant tel qu'un éther (tétrahydrofuranne, dioxanne par exemple) à une température comprise entre -20 et 20°C, puis d'un dérivé de quinoléine de formule générale (VII), par analogie avec les méthodes décrites par Suzuki et al., Pure and Appl. Chem., 57, 1749 (1985) et élimination du radical Rz protecteur de l'amino. La réaction s'effectue généralement en présence d'un sel de palladium (chlorure de palladium diphénylphosphinoferrocène par exemple) et d'une base comme le phosphate de potassium à une température comprise entre 20°C et la température de reflux du solvant.

L'élimination du radical Rz s'effectue selon les méthodes connues et citées ci avant, citées dans les exemples, ou décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le dérivé de la pipéridine de formule générale (VIII) peut être préparé par réaction de Wittig, par condensation d'un ylure de phosphore sur un dérivé de pipéridine de formule générale : 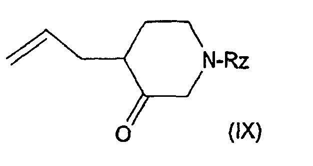
dans laquelle Rz est défini comme ci-dessus.

On opère avantageusement au moyen de (triphénylphosphoranylidène) acétate de méthyle, dans un solvant comme par exemple le toluène, à une température comprise entre 20 et 110°C.

Le dérivé d'oxo-3 pipéridine de formule générale (IX) peut être préparé selon ou par analogie avec la méthode décrite par Y. Takeuchi et coll., Synthesis, 10, 1814 (1999).

Les dérivés de la quinoléine de formule générale (VII) peuvent être préparés selon la méthode décrite dans la demande de brevet WO200240474-A2.

Le dérivé de quinolyl propyl pipéridine de formule générale (II) pour lequel R'₂ est un radical carboxy protégé et R'₁ₐ et R_{1b} sont des atomes d'hydrogène peut être préparé à partir du dérivé correspondant pour lequel R'₂ est carboxyméthyle protégé, par réduction de ce radical en un radical hydroxyéthyle, transformation en un dérivé p-toluènesulfonyloxyéthyle, puis transformation de ce dérivé en dérivé vinylique par réaction d'élimination suivie de l'oxydation du dérivé obtenu en dérivé carboxy et de l'introduction du groupement protecteur sur le radical carboxy ainsi obtenu.

La réduction de l'acide protégé en un radical hydroxyéthyle s'effectue selon les méthodes habituelles qui n'altèrent pas le reste de la molécule, notamment on opère par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.
La transformation du dérivé hydroxyéthyle en un dérivé p-toluènesulfonyloxyéthyle s'effectue notamment selon la méthode décrite par L.F. Fieser et M. Fieser, Reagents for Organic Synthesis, vol 1, 1179 (1967), à partir du chlorure de p-toluènesulfonyle en présence d'une base comme une amine tertiaire (par exemple la triéthylamine) ou aromatique (par exemple la pyridine), dans un solvant halogéné (dichlorométhane par exemple) ou sans solvant, à une température comprise entre 0 et 50°C.
La transformation du dérivé p-toluènesulfonyloxyéthyle en dérivé vinylique s'effectue par réaction d'élimination, notamment selon la méthode décrite par A. Sharma et coll., Org. Prep Proced. Int., 25(3), 330-333 (1993), en présence-d'une base comme par exemple le t.butylate de potassium dans un solvant tel que le diméthylsulfoxyde par exemple, à une température comprise entre 20 et 100°C.

La transformation du dérivé vinylique en un dérivé carboxy s'effectue par les méthodes d'oxydation décrites dans la littérature, notamment par le méta periodate de sodium en présence d'hydrate de trichlorure de ruthénium, dans un mélange de solvants comme par exemple le mélange eau/acétonitrile, à une température comprise entre 20 et 60°C.

Selon une alternative, le dérivé de quinolyl propyl pipéridine de formule générale (II), pour lequel R'₁ₐ et R_{1b} sont des atomes d'hydrogène peut être préparé par condensation d'un dérivé de la quinoléine de formule générale (VII) tel que défini précédemment, sur un dérivé de pipéridine de formule générale : 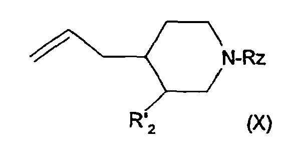
dans laquelle Rz et R'₂ sont définis comme précédemment, pour obtenir un dérivé de formule générale XI 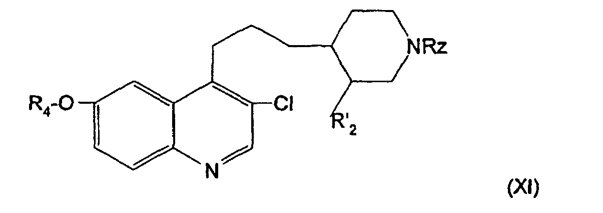
puis élimine le radical Rz protecteur d'amino.

La réaction s'effectue dans des conditions analogues aux conditions décrites pour la réaction du dérivé de quinoléine de formule générale (VII) et du dérivé de la pipéridine de formule générale (VIII).

L'élimination du radical Rz s'effectue selon les méthodes connues et citées ci avant.

Selon l'invention, les dérivés correspondant à ceux de formule générale (XI) ci-dessus, dans laquelle R'₂ représente un radical carboxy protégé peuvent être transformés en dérivés dans lesquels R'₂ représente un radical carboxyméthyle dans des conditions analogues à celles décrites plus haut, c'est-à-dire par réduction du carboxy protégé en hydroxyméthyle et transformation de celui-ci en carboxyméthyle.

Le dérivé de pipéridine de formule générale (X), peut être préparé par deoxygénation radicalaire à l'aide de l'hydrure de tributylétain en présence de 2,2'-azobisisobutyronitrile (AIBN) d'un composé de formule générale (XII) : 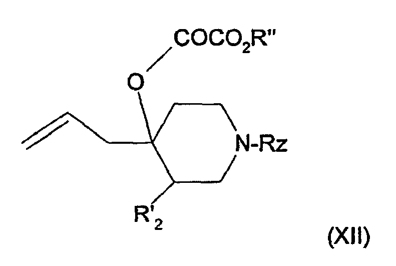
dans laquelle R" représente un radical alkyle, de préférence méthyle, R'₂ et Rz sont définis comme précédemment.

La réaction de déoxygénation radicalaire s'effectue avec de l'hydrure de tributylétain en présence d'AIBN dans un solvant inerte comme le toluène ou le benzène à une température comprise entre 20°C et le reflux par analogie à la méthode décrite dans J. Org. Chem., 1996, 61, 7189.

Le dérivé de pipéridine de formule générale (XII) peut être obtenu par action d'un halogénure d'alkyloxalyle tel que le chlorure de méthyloxalyle sur un dérivé de formule générale (XIII) : 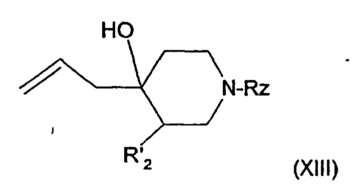
dans laquelle R'₂ et Rz sont définis comme précédemment.

Cette réaction s'effectue en présence d'une base comme la 4-diméthylaminopyridine dans un solvant inerte comme l'acétonitrile ou le dichlorométhane à une température comprise entre 0°C et 50°C par analogie à la méthode décrite dans J. Org. Chem., 1996, 61, 7189.

Le dérivé de pipéridine de formule générale (XIII), dans laquelle R'₂ est un radical carboxy protégé et Rz est défini comme précédemment peut être obtenu par une réaction d'allylation du cétoester de formule générale (XIV) 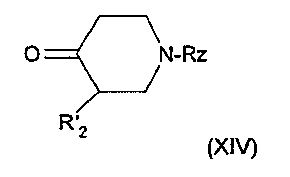
pour laquelle R'₂ et Rz sont définis comme précédemment.

Lorsque R'₂ représente un radical carboxy protégé, cette réaction d'allylation s'effectue soit à l'aide du bromure d'allyle, du zinc et du chlorure d'ammonium dans un solvant inerte comme le tétrahydrofurane ou le dioxane à une température comprise entre 20°C et le reflux du solvant par analogie à la méthode décrite dans J. Chem. Soc. Chem. Comm., 1994, 1217, soit à l'aide du bromure d'allyle en présence d'indium dans un mélange d'alcool, comme le méthanol ou l'éthanol et d'eau à une température comprise entre 20°C et 70°C par analogie à la méthode décrite dans Tetrahedron Letters, 1998, 54, 2347.

Lorsque R'₂ représente un radical carboxy méthyle protégé, l'alkylation peut être effectuée par une réaction de type Grignard, en mettant en jeu un réactif organométallique approprié.

Les composés de formule générale (XIV) sont connus ou sont préparables par des procédés connus, par exemple à partir de 4-oxo-3-piperidinecarboxylate d'alkyle, de préférence de 4-oxo-3-piperidinecarboxylate de méthyle, par application ou adaptation de la méthode décrite dans Tetrahedron Letters, 1991, 32, 3643 ou à partir de 4-oxo-3-pipéridine acétate d'alkyle ou d'acide 4-oxo-3-pipéridine acétique, dont l'atome d'azote est protégé. De tels dérivés sont décrits par exemple dans Chem. Pharm. Bull (1983), 31 (11), 4135-8 ou dans les demandes japonaises JP 54098771 ou 56038147.

Les différents intermédiaires de type quinolyl propyl pipéridine pour lesquels R₄ représente alcényl-CH₂-, alcynyl-CH₂-, cycloalcoyle ou cycloalcoyl alcoyle peuvent être obtenus par analogie avec la préparation des intermédiaires pour lesquels R₄ est alcoyle, par action du dérivé halogéné correspondant sur le dérivé de quinoléine hydroxylé en position 6.

Il est entendu que les dérivés de formule générale (I), mais aussi les intermédiaires de formules (II), (III), (IV) ainsi que leurs intermédiaires de préparation présentent une isomérie «cis/trans» au niveau des substituants en position 3 et 4 de la pipéridine. Les dérivés de configuration «trans» peuvent être obtenus à partir des dérivés de configuration «cis» selon ou par analogie avec la méthode décrite dans la demande internationale WO 99/37635, ou à partir d'intermédiaires existant sous forme de mélanges, après séparation selon les méthodes connues.

Les dérivés de quinolyl propyl pipéridine de formule générale (I) peuvent être purifiés, le cas échéant, par des méthodes physiques telles que la cristallisation ou la chromatographie.

Par ailleurs, il est également entendu que d'une part pour les composés de formule générale (I) lorsque R_{1b} est un atome d'hydrogène et R₁ₐ est autre que l'atome d'hydrogène et d'autre part pour les composés de formule générale (XII) et (XIII), il existe également des formes énantiomères et diastéréoisomères, lesquelles formes ainsi que leurs mélanges entrent dans le cadre de la présente invention. Ces derniers peuvent la cas échéant être séparés notamment par chromatographie sur silice ou par Chromatographie Liquide Haute Performance (CLHP). De même, les dérivés cis et trans peuvent être séparés par chromatographie sur silice ou par Chromatographie Liquide Haute Performance (CLHP).

Les dérivés de quinolyl propyl pipéridine de formule générale (I) peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés de quinolyl propyl pipéridine de formule générale (I) portant un radical carboxy peuvent être transformés à l'état de sels métalliques ou en sels d'addition avec les bases azotées selon les méthodes connues en soi. Ces sels entrent également dans le cadre de la présente invention. Les sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les dérivés de quinolyl propyl pipéridine selon l'invention sont des agents antibactériens particulièrement intéressants.

In vitro, sur germes gram positifs les dérivés de quinolyl propyl pipéridine selon l'invention se sont montrés actifs à des concentrations comprises entre 0,03 et 4 µg/ml sur *Staphylococcus aureus* AS5155 résistante à la méticilline, également à des concentrations comprises entre 0,06 et 8 µg/ml sur *Streptococcus pneumoniae* 6254-01 et à des concentrations comprises entre 0,06 et 64 µg/ml sur *Enterococcus faecium* H983401 et sur germes gram négatifs, ils se sont montrés actifs à des concentrations comprises entre 0,12 et 32 µg/ml sur *Moraxella catharrhalis* IPA152 ; in vivo, ils se sont montrés actifs sur les infections expérimentales de la souris à *Staphylococcus aureus* IP8203 à des doses comprises entre 12 et 150 mg/kg par voie sous cutanée (DC₅₀) et pour certains d'entre eux à des doses comprises entre 26 et 150 mg/kg par voie orale.

En outre, les produits selon l'invention sont particulièrement intéressants du fait de leur faible toxicité. Aucun des produits n'a manifesté de toxicité à la dose de 100 mg/kg par voie sous cutanée chez la souris.

Ces propriétés rendent aptes lesdits produits, ainsi que leurs sels d'acides et de bases pharmaceutiquement acceptables, à être utilisés comme médicaments dans le traitement des affections à germes sensibles provoquées par des bactéries à gram^{⊕} et notamment dans celui des staphylococcies, telles que septicémies à staphylocoques, staphylococcies malignes de la face ou cutanée, pyodermites, plaies septiques ou suppurantes, anthrax, phlegmons, érysipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments destinés au traitement des infections bactériennes chez l'homme ou l'animal, les composés de formule (I) tels que définis ci-dessus ainsi que leurs sels pharmaceutiquement acceptables, et notamment les composés préférés mentionnés plus haut.

La présente invention concerne également les compositions pharmaceutiques contenant au moins un dérivé de quinolyl propyl pipéridine selon l'invention, le cas échéant sous forme de sel, à l'état pur ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administrations orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions pour administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de quinolyl propyl pipéridine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

L'exemple suivant illustre une composition selon l'invention.

On prépare selon la technique habituelle une composition liquide destinée à l'usage parentéral comprenant :
- Acide (3R,4R) -4-[3-(S)-hydroxy-(3-chloro-6-méthoxyquinolin-4-yl)propyl]-1-[2-(2,5-difluorophénylthio)éthyl]pipéridine-3-carboxylique ................................................................................................... 1 g
- Glucose ........................................................................................................... qsp 2,5%
- hydroxyde de sodium.................................................................................... qsp pH = 4-4,5
- eau ppi.............................................................................................................. qsp 20 ml

L'invention a enfin pour objet, à titre de produits industriels nouveaux et notamment à titre de produits intermédiaires nécessaires à la préparation des produits de formule (I) :
- les produits de formule (II) telle que définie plus haut ;
- les produits de formule (A)

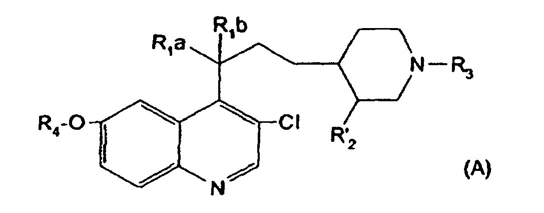

dans laquelle R₁ₐ, R_{1b}, R'₂, R₃ et R₄ sont tels que définis plus haut, correspondant aux produits de formule (III) ou obtenus intermédiairement à l'issue des différents traitements effectués sur les produits de formule (III) ;
- les produits de formule (IV) telle que définie plus haut ;
- les produits de formule (VI) telle que définie plus haut ;
- les produits de formule (XI) telle que définie plus haut.

Parmi les produits selon l'invention, plus particulièrement intéressants sont les dérivés de quinolyl propyl pipéridine cités ci-après, et notamment ceux décrits dans les exemples, à titre non limitatif :
- Acide (3RS, 4RS) ou (3SR, 4RS)-4-[3-(3-chloro-6-méthoxyquinolin-4-yl)propyl]-1-(2-phénylthioéthyl)pipéridine-3-carboxylique
- Acide (3RS, 4RS) ou (3SR, 9RS)-9-[3-(3-chloro-6-méthoxyquinolin-9-yl)propyl]-1-[2-(3-fluorophénylthio)éthyl]pipéridine-3-carboxylique
- Acide (3RS,4RS) ou (3SR,4RS)-4-[3-(3-chloro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(3-chloro-6-méthoxyquinolin-4-yl)propyl]-l-[2-(3,5-difluorophénylthio)éthyl]pipéridine-3-carboxylique
- Acide (3RS, 4RS) ou (3SR,4RS)-4-[3-(3-chloro-6-méthoxyquinolin-4-yl)propyl]-1-[2-(2,3,5-trifluorophénylthio)éthyl]pipéridine-3-carboxylique
- Acide (3RS,4RS) ou (3SR, 4RS)-4-[3-(3-chloro-6-méthoxyquinolin-4-yl) propyl]-1-[2-(n-propylthio)éthyl]pipéridine-3-carboxylique
- Acide (3RS,4RS) ou (3SR,4RS)-4-[3-(3-chloro-6-méthoxyquinolin-4-yl)propyl]-1-[2-(n-butylthio)éthyl]pipéridine-3-carboxylique
- Acide (3RS,4RS) ou (3SR,4RS)-4-[3- (3-chloro-6-méthoxyquinolin-4-yl)propyl]-1-[2-(cyclopropylthio)éthyl]pipéridine-3-carboxylique
- Acide (3RS,4RS) ou (3SR,4RS)-4-[3-(3-chloro-6-méthoxyquinolin-4-yl)propyl]-1-[2-(cyclobutylthio)éthyl]pipéridine-3-carboxylique
- Acide (3RS,4RS) ou (3SR,4RS)-4-[3-(3-chloro-6-méthoxyquinolin-4-yl)propyl]-1-[2-(cyclopentylthio)éthyl]pipéridine-3-carboxylique
- Acide (3RS, 4RS) ou (3SR,4RS)-4-[3-(3-chloro-6-méthoxyquinolin-4-yl)propyl]-1-[2-(cyclohexylthio)éthyl]pipéridine-3-carboxylique
- Acide(3RS,4RS) ou (3SR,4RS)-4-[3-(3-chloro-6-méthoxyquinolin-4-yl)propyl]-1-[2-(thien-2-yl)thioéthyl]-pipéridine-3-carboxylique
- Acide (3RS,4RS) ou (3SR,4RS)-4-[3-(3-chloro-6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-(3-chloro-6-méthoxyquinolin-4-yl)propyl]-2-[2-(4-fluorothien-2-yl)thioéthyl]pipéridine-3-carboxylique
- Acide (3RS,4RS) ou (3SR,4RS)-4-[3-(3-chloro-6-méthoxyquinolin-4-yl)propyl]-1-[2-(3-fluorothien-2-yl)thioéthyl]pipéridine-3-carboxylique
- Acide (3RS,4RS) ou (3SR,4RS)-4-[3-(3-chloro-6-méthoxyquinolin-4-yl)propyl]-1-[2-(thien-3-yl)thioéthyl]pipéridine-3-carboxylique
- Acide (3RS,4RS) ou (3SR,4RS)-4-[3-(3-chloro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(3-chloro-6-méthoxyquinolin-4-yl)propyl]-1-[2-(pyridin-2-yl)thioéthyl]pipéridine-3-carboxylique
- Acide (3RS,4RS) ou (3SR, 4RS)-4-[3-(3-chloro-6-méthoxyquinolin-4-yl)propyl]-1-[2-(4-fluoropyridin-2-yl)thioéthyl]pipéridine-3-carboxylique
- Acide (3RS,4RS) ou (3SR,4RS)-4-[3-(3-chloro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-(3-chloro-6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(2,5-difluoro-phényl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(thien-2-yl)-prop-2-ynyl]-pipéridine-3-carboxylique
- Acide (3RS,4RS) ou (3R,4RS)-4-[3-(3-chloro-6-méthoxyquinolin-4-yl)propyl]-1-[3-(5-fluorothien-2-yl)-prop-2-ynyl]-pipéridine-3-carboxylique
- Acide (3RS,4RS) ou (3SR, 4RS)-4-[3-(3-chloro-6-méthoxyquinolin-4-yl)propyl]-2-[3-(4-fluorothien-2-yl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-(3-chloro-6-méthoxyquinolin-4-yl)propyl]-1-[3-(thien-3-yl)-prop-2-ynyl]-pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-(2-phénylthioéthyl)pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-[2-(3-fluorophénylthio)éthyl]pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(2,3,5-trifluorophénylthio)éthyl]pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-[2-(n-propylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(n-butylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(cyclopropylthio)éthyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(cyclobutylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(cyclopentylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(cyclohexylthio)éthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(thien-2-yl)thioéthyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(5-fluorothien-2-yl)-thioéthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(4-fluorothien-2-yl)-thioéthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(3-fluorothien-2-yl)-thioéthyl]pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-[2-(thien-3-yl)thioéthyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(pyridin-2-yl)thioéthyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(4-fluoropyridin-2-yl)thioéthyl]pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[2-(3-fluoro-pyridin-2-yl)thioéthyl]pipéridine-3-carboxylique
- Acide (3RS, 4RS) ou (3SR,4RS)-4-[3-(R,S)-hydroxy-3-(3-chloro-6-méthoxyquinolin-4-yl)propyl]-1-[3-(3-fluoro-phényl)-prop-2ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(2,5-difluoro-phényl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(thien-2-yl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(5-fluorothien-2-yl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(4-fluorothien-2-yl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-(R,S)-hydroxy-3-(3-chloro-6-méthoxyquinolin-4-yl)propyl]-1-[3-(thien-3-yl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-(2-phénylthioéthyl)pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(3-fluorophénylthio)-éthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(2,5-difluorophénylthio)-éthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(3,5-difluorophénylthio)-éthyl]pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[2-(2,3,5-trifluorophénylthio) - éthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(n-propylthio)éthyl]-pipéridine-3-carboxylique
- Acide (3RS,4RS) ou (3SR,4RS)-4-[3-(R,S)-fluoro-3-(3-chloro-6-mëthoxyquinolin-4-yl)propyl]-1-[2-(n-butylthio)éthyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(cyclopropylthio)éthyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(cyclobutylthio)éthyl]-pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[2-(cyclopentylthio)éthyl]-pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[2-(cyclohexylthio)éthyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(thien-2-yl)thioéthyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(4-fluorothien-2-yl)thioéthyl]pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[2-(3-fluorothien-2-yl)thioéthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(thien-3-yl)thioéthyl]-pipéridine-3-carboxylique
- Acide (3RS,4RS) ou (3SR, 4RS)-4-[3-(R,S)-fluoro-3-(3-chloro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(pyridin-2-yl)thioéthyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[2-(4-fluoropyridin-2-yl)thioéthyl]pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(2,5-difluoro-phényl)-prop-2-ynyl]-pipéridine-3-carboxylique
- Acide (3RS, 4RS) ou (3SR,4RS)-4-[3-(R,S)-fluoro-3-(3-chloro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(thien-2-yl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(5-fluorothien-2-yl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(4-fluorothien-2-yl)-prop-2-ynyl]-pipéridine-3-carboxylique
- Acide (3RS,4RS) ou (3SR,4RS)-4-[3-(R,S)-fluoro3-(3-chloro-6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-(R,S)-fluoro-3-(3-chloro-6-méthoxyquinolin-4-yl)propyl]-1-[3-(thien-3-yl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-(2-phénylthioéthyl)pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(3-fluorophénylthio)-éthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(2,5-difluorophénylthio)-éthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(3,5-difluorophénylthio)-éthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(2,3,5-trifluorophénylthio)-éthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(n-propylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(n-butylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(cyclopropylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(cyclobutylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(cyclopentylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(cyclohexylthio)éthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(thien-2-yl)thioéthyl]-pipéridine-3-carboxylique
- Acide (3RS, 4RS) ou (3SR,4RS)-4-[3-(R,S)- amino-3-(3-chloro-6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(4-fluorothien-2-yl)thioéthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(3-fluorothien-2-yl)thioéthyl]pipéridine-3-carboxylique
- Acide (3RS, 4RS) ou (3SR, 4RS)-4-[3-(R, S)-amino-3-(3-chloro-6-méthoxyquinolin-4-yl)propyl]-1-[2-(thien-3-yl)thioéthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(pyridin-2-yl)thioéthyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[2-(4-fluoropyridin-2-yl)thioéthyl]pipéridine-3-carboxylique
- Acide (3RS, 4RS) ou (3SR,4RS)-4-[3-(R,S)-amino-3-(3-chloro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-(R,S)-amino-3-(3-chloro-6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-2-[3-(2,5-difluoro-phényl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[3-(thien-2-yl)-prop-2-ynyl]-pipéridine-3-carboxylique
- Acide (3RS, 4RS) ou (3SR,4RS)-4-[3-(R,S)-amino-3-(3-chloro-6-méthoxyquinolin-4-yl)propyl]-1-[3-(5-fluorothien-2-yl)-prop-2-ynyl]-pipéridine-3-carboxylique
- Acide (3RS, 4RS) ou (3SR,4RS)-4-[3-(R,S)-amino-3-(3-chloro-6-méthoxyquinolin-4-yl)propyl]-1-[3-(4-fluorothien-2-yl)-prop-2-ynyl]-pipéridine-3-carboxylique
- Acide (3RS, 4RS) ou (3SR, 4RS)-4-[3-(R, S)-amino-3-(3-chloro-6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-(R,S)-amino-3-(3-chloro-6-méthoxyquinolin-4-yl)propyl]-1-[3-(thien-3-yl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-(2-phénylthioéthyl)pipéridine-3-carboxylique
- Acide (3RS,4RS) ou (3SR,4RS)-4-[3-oxo-3-(3-chloro-6-méthoxyquinolin-4-yl)propyl]-1-[2-(3-fluorophénylthio)-éthyl]pipéridine-3-carboxylique
- Acide (3RS,4RS) ou (3SR,4RS)-4-[3-oxo-3-(3-chloro-6-méthoxyquinolin-4-yl)propyl]-1-[2-(2,5-difluorophénylthio)-éthyl]pipéridine-3-carboxylique
- Acide (3RS, 4RS) ou (3SR,4RS)-4-[3-oxo-3-(3-chloro-6-méthoxyquinolin-4-yl)propyl]-1-[2-(3,5-difluorophénylthio)-éthyl]pipéridine-3-carboxylique
- Acide (3RS,4RS) ou (3SR,4RS)-4-[3-oxo-3-(3-chloro-6-méthoxyquinolin-4-yl)propyl]-2-[2-(2,3,5-trifluorophénylthio)éthyl]pipéridine-3-carboxylique
- Acide (3RS,4RS) ou (3SR,4RS)-4-[3-oxo-3-(3-chloro-6-méthoxyquinolin-4-yl)propyl]-1-[2-(n-propylthio)éthyl]pipéridine-3-carboxylique
- Acide (3RS,4RS) ou (3SR,4RS)-4-[3-oxo-3-(3-chloro-6-méthaxyquinolin-4-yl)propyl]-1-[2-(n-butylthio)éthyl]pipéridine-3-carboxylique
- Acide (3RS,4RS) ou (3SR,4RS)-4-[3-oxo-3-(3-chloro-6-méthoxyquinolin-4-yl)propyl]-2-[2-(cyclopropylthio)éthyl]pipéridine-3-carboxylique
- Acide (3RS, 4RS) ou (3SR,4RS)-4-[3-oxo-3-(3-chloro-6-méthoxyquinolin-4-yl)propyl]-1-[2-(cyclobutylthio)éthyl] pipéridine-3-carboxylique
- Acide (3RS,4RS) ou (3SR,4RS)-4-[3-oxo-3-(3-chloro-6-méthoxyquinolin-4-yl)propyl]-1-[2-(cyclopentylthio)éthyl]pipéridine-3-carboxylique
- Acide (3RS,4RS) ou (3SR,4RS)-4-[3-oxo-3-(3-chloro-6-méthoxyquinolin-4-yl)propyl]-1-[2-(cyclohexylthio)éthyl]pipéridine-3-carboxylique
- Acide (3RS,4RS) ou (3SR,4RS)-4-[3-oxo-3-(3-chloro-6-méthoxyquinolin-4-yl)propyl]-1-[2-(thien-2-yl)thioéthyl]-pipéridine-3-carboxylique
- Acide (3RS,4RS) ou (3SR,4RS)-4-[3-oxo-3-(3-chloro-6-méthoxyquinolin-9-yl)propyl]-1-[2-(5-fluorothien-2-yl)thioéthyl]pipéridine-3-carboxylique
- Acide (3RS,4RS) ou (3SR,4RS) -4- [3-oxo-3- (3-chloro-6-méthoxyquinolin-4-yl)propyl]-1-[2-(4-fluorothien-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(3-fluorothien-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(thien-3-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(pyridin-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(4-fluoropyridin-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-oxo-3-(3-chloro-6-méthoxyquinolin-4-yl)propyl]-1-[3-(3-fluoro-phényl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(2,5-difluoro-phényl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(3,5-difluoro-phényl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(2,3,5-trifluoro-phényl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(thien-2-yl)-prop-2-ynyl]-pipéridine-3-carboxylique
- Acide (3RS,4RS) ou (3SR,4RS)-43-oxo-3-(3-chloro-6-méthoxyquinolin-4-yl)propyl]-1-[3-(5-fluorothien-2-yl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(4-fluorothien-2-yl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(3-fluorothien-2-yl)-prop-2-ynyl]-pipéridine-3-carboxylique
- Acide (3RS,4RS) ou (3SR,4RS)-4-[3-oxo-3-(3-chloro-6-méthoxyquinolin-4-yl)propyl]-1-[3-(thien-3-yl)-prop-2-ynyl]-pipéridine-3-carboxylique
- Acide (3RS,4RS) ou (3SR,4RS)-4-[3-(3-chloro-6-méthoxyquinolin-4-yl)propyl]-1-(2-phénylthioéthyl)pipéridine-3-acétique
- Acide (3RS,4RS) ou (3SR,4RS)-4-[3-(3-chloro-6-méthoxyquinolin-4-yl) propyl]-1-[2-(3-fluorophénylthio)éthyl]pipéridine-3-acétique
- Acide (3RS,4RS) ou (3SR,4RS)-4-[3-(3-chloro-6-méthoxyquinolin-4-yl)propyl]-1-[2-(2,5-difluorophénylthio)éthyl]pipéridine-3-acétique
- Acide (3RS,4RS) ou (3SR,4RS)-4-[3-(3-chloro-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(3-chloro-6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-(3-chloro-6-méthoxyquinolin-4-yl)propyl]-1-[2-(n-propylthio)éthyl]pipéridine-3-acétique
- Acide (3RS,4RS) ou (3SR,4RS)-4-[3-(3-chloro-6-méthoxyquinolin-4-yl)propyl]-1-[2-(n-butylthio)éthyl]pipéridine-3-acétique
- Acide (3RS,4RS) ou (3SR,4Rs)-4- [3-(3-chloro-6-méthoxyquinolin-4-yl)propyl]-1-[2-(cyclopropylthio)éthyl]pipéridine-3-acétique
- Acide (3RS,4RS) ou (3SR,4RS)-4-[3-(3-chloro-6-méthoxyquinolin-4-yl)propyl]-1-[2-(cyclobutylthio)éthyl]pipéridine-3-acétique
- Acide (3RS,4RS) ou (3SR,4RS)-4-[3-(3-chloro-6-méthoxyquinolin-4-yl)propyl]-1-[2-(cyclopentylthio)éthyl]pipéridine-3-acétique
- Acide (3RS,4RS) ou (3SR,4RS)-4-[3-(3-chloro-6-méthoxyquinolin-4-yl)propyl]-1-[2-(cyclohexylthio)éthyl]pipéridine-3-acétique
- Acide (3RS,4RS) ou (3SR,4RS)-4-[3-(3-chloro-6-méthoxyquinolin-4-yl)propyl]-1-[2-(thien-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(5-fluorothien-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(4-fluorothien-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(3-fluorothien-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(thien-3-yl)thioéthyl]pipéridine-3-acétique
- Acide (3RS,4RS) ou (3SR,4RS)-4-[3-(3-chloro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(pyridin-2-yl)thioéthyl]pipéridine-3-acétique
- Acide (3RS,4RS) ou (3SR,4RS)-4-[3-(3-chloro-6-méthoxyquinolin-4-yl)propyl]-1-[2-(4-fluoropyridin-2-yl)thioéthyl]pipéridine-3-acétique
- Acide (3RS,4RS) ou (3SR,4RS)-4-[3-(3-chloro-6-méthoxyquinolin-4-yl) propyl] -1- [2- (3-fluoro-pyridin-2-yl) thioéthyl]pipéridine-3-acétique
- Acide (3RS,4RS) ou (3SR,4RS)-4-[3-(3-chloro-6-méthoxyquinolin-4-yl)propyl]-1-[3-(3-fluoro-phényl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(thien-2-yl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(S-fluorothien-2-yl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-(3-chloro-6-méthoxyquinolin-4-yl)propyl]-1-[3-(3-fluorothien-2-yl)-prop-2-ynyl]-pipéridine-3-acétique
- Acide (3RS,4RS) ou (3SR, 4RS)-4-[3-(3-chloro-6-méthoxyquinolin-4-yl)propyl]-1-[3-(thien-3-yl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-(2-phénylthioéthyl)pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(3-fluorophénylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(2,5-difluorophénylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(n-propylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-2-[2-(n-butylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(cyclopropylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(cyclobutylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(cyclopentylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(cyclohexylthio)éthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(thien-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-2-[2-(5-fluorothien-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(4-fluorothien-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-2-[2-(3-fluorothien-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(thien-3-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(pyridin-2-yl)thioéthyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[2 -(4-fluoropyridin-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[2-(3-fluoro-pyridin-2-yl)thioéthyl]pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(3-fluoro-phényl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(thien-2-yl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(5-fluorothien-2-yl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(3-fluorothien-2-yl)-prop-2-ynyl]-pipéridine-3-acétique
- Acide (3RS,4RS) ou (3SR,4RS)-4-[3-(R,S)-hydroxy-3-(3-chloro-6-méthoxyquinolin-4-yl)propyl]-1-[3-(thien-3-yl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-(2-phénylthioéthyl)pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(3-fluorophénylthio)éthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(2,5-difluorophénylthio)-éthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(3,5-difluorophénylthio)-éthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(2,3,5-trifluorophénylthio)-éthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(n-propylthio)éthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(n-butylthio)éthyl]pipéridine-3-acétique
- Acide (3RS,4RS) ou (3SR,4RS)-4-[3-(R,S)-fluoro-3-(3-chloro-6-méthoxyquinolin-4-yl)propyl]-|-[2-(cyclopropylthio)éthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(cyclobutylthio)éthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(cyclopentylthio)éthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(cyclohexylthio)éthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(thien-2-yl)thioéthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(5-fluorothien-2-yl)-thioéthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(4-fluorothien-2-yl)-thioéthyl]pipéridine-3-acétique
- Acide (3RS, 4RS) ou (3SR,4RS)-4-[3-(R,S)-fluoro-3-(3-chloro-6-méthoxyquinolin-4-yl)propyl]-1-[2-(3-fluorothien-2-yl)-thioéthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(thien-3-yl)thioéthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(pyridin-2-yl)thioéthyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(4-fluoropyridin-2-yl)thioéthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[2-(3-fluoro-pyridin-2-yl)thioéthyl]pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(3-fluoro-phényl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(thien-2-yl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(5-fluorothien-2-yl)-prop-2-ynyl] -pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-(R,S)-fluoro3-(3-chloro-6-méthoxyquinolin-4-yl)propyl]-1-[3-(3-fluorothien-2-yl)-prop-2-ynyl]-pipéridine-3-acétique
- Acide (3RS,4RS) ou (3SR,4RS)-4-[3-(R,S)-fluoro-3-(3-chloro-6-méthoxyquinolin-4-yl)propyl]-1-[3-(thien-3-yl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-(2-phénylthioéthyl)pipéridine-3-acétique
- Acide (3RS,4RS) ou (3SR,4RS)-4-[3-(R,S)-amino-3-(3-chloro-6-méthoxyquinolin-4-yl)propyl]-2-[2-(3-fluorophénylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(2,5-difluorophénylthio)-éthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(3,5-difluorophénylthio)-éthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(2,3,5-trifluorophénylthio)-éthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(n-propylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(n-butylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(cyclopropylthio)éthyl]-pipéridine-3-acétique
- Acide (3RS,4RS) ou (3SR,4RS)-4-[3-(R, S)-amino-3-(3-chloro-6-méthoxyquinolin-4-yl)propyl]-1-[2-(cyclobutylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(cyclopentylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(cyclohexylthio)éthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(thien-2-yl)thioéthyl]-pipéridine-3-acétique
- Acide (3RS,4RS) ou (3SR,4RS)-4-[3-(R,S)- amino-3-(3-chloro-6-méthoxyquinolin-4-yl)propyl]-1-[2-(5-fluorothien-2-yl)-thioéthyl]pipéridine-3-acétigue
- Acide (3RS,4RS) ou (3SR,4RS)-4-[3-(R,S)-amino-3-(3-chloro-6-méthoxyquinolin-4-yl)propyl]-1-[2-(4-fluorothien-2-yl)-thioéthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(3-fluorothien-2-yl)-thioéthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(thien-3-yl)thioéthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(pyridin-2-yl)thioéthyl]-pipéridine-3-acétique
- Acide (3RS,4RS) ou (3SR,4RS)-4-[3-(R,S)-amino-3-(3-chloro-6-méthoxyquinolin-4-yl)propyl]-1-[2-(4-fluoropyridin-2-yl)thioéthyl]pipéridine-3-acétique
- Acide (3RS,4RS) ou (3SR,4RS)-4-[3-(R,S)-amino-3-(3-chloro-6-méthoxyquinolin-4-yl)propyl]-1-[2-(3-fluoro-pyridin-2-yl)-thioéthyl]pipéridine-3-acétique
- Acide (3RS,4RS) ou (3SR,4RS)-4-[3-(R,S)-amino-3-(3-chloro-6-méthoxyquinolin-4-yl)propyl]-2-[3-(3-fluoro-phényl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(thien-2-yl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(5-fluorothien-2-yl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(3-fluorothien-2-yl)-prop-2-ynyl]-pipéridine-3-acétique
- Acide (3RS,4RS) ou (3SR,4RS)-4-[3-(R,S)-amino-3-(3-chloro-6-méthoxyquinolin-4-yl)propyl]-1-[3-(thien-3-yl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-(2-phénylthioéthyl)pipéridine-3-acétique
- Acide (3RS,4RS) ou (3SR,4RS)-4-[3-oxo-3-(3-chloro-6-méthoxyquinolin-4-yl)propyl]-1-[2-(3-fluorophénylthio)-éthyl]pipéridine-3-acétique
- Acide (3RS,4RS) ou (3SR,4RS)-4-[3-oxo-3-(3-chloro-6-méthoxyquinolin-4-yl)propyl]-1-[2-(2,5-difluorophénylthio)-éthyl]pipéridine-3-acétique
- Acide (3RS,4RS) ou (3SR,4RS)-4-[3-oxo-3-(3-chloro-6-méthoxyquinolin-4-yl)propyl]-1-[2-(3,5-difluorophénylthio)-éthyl]pipéridine-3-acétique
- Acide (3RS,4RS) ou (3SR,4RS)-4-[3-oxo-3-(3-chloro-6-méthoxyquinolin-4-yl)propyl]-1-[2-(2,3,5-trifluorophénylthio)-éthyl]pipéridine-3-acétique
- Acide (3RS,4RS) ou (3SR,4RS)-4-[3-oxo-3-(3-chloro-6-méthoxyquinolin-4-yl)propyl]-1-[2-(n-propylthio)éthyl]pipéridine-3-acétique
- Acide (3RS,4RS) ou (3SR,4RS)-4-[3-oxo-3-(3-chloro-6-méthoxyquinolin-4-yl)propyl]-1-[2-(n-butylthio)éthyl]pipéridine-3-acétique
- Acide (3RS,4RS) ou (3SR,4RS)-4-[3-oxo-3-(3-chloro-6-méthoxyquinolin-4-yl)propyl]-1-[2-(cyclopropylthio)éthyl]pipéridine-3-acétique
- Acide (3RS,4RS) ou (3SR,4RS)-4-[3-oxo-3-(3-chloro-6-méthoxyquinolin-4-yl)propyl]-1-[2-(cyclobutylthio)éthyl]pipéridine-3-acétique
- Acide (3RS,4RS) ou (3SR,4RS)-4-[3-oxo-3-(3-chloro-6-méthoxyquinolin-4-yl)propyl]-1-[2-(cyclopentylthio)éthyl]pipéridine-3-acétique
- Acide (3RS,4RS) ou (3SR,4RS)-4-[3-oxo-3-(3-chloro-6-méthoxyquinolin-4-yl)propyl]-1-[2-(cyclohexylthio)éthyl]pipéridine-3-acétique
- Acide (3RS, 4RS) ou (3SR,4RS)-4-[3-oxo-3-(3-chloro-6-méthoxyquinolin-4-yl)propyl]-1-[2-(thien-2-yl)thioéthyl]-pipéridine-3-acétique
- Acide (3RS,4RS) ou (3SR,4RS)-4-[3-oxo-3-(3-chloro-6-méthoxyquinolin-4-yl)propyl]-1-[2-(5-fluorothien-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[2-(4-fluorothien-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[2-(3-fluorothien-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[2-(thien-3-yl)thio-éthyl]-pipéridine-3-acétique
- Acide (3RS,4RS) ou (3SR,4RS)-4-[3-oxo-3-(3-chloro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[2-(pyridin-2-yl)thioéthyl]-pipéridine-3-acétique
- Acide (3RS,4RS) ou (3SR,4RS)-4-[3-oxo-3-(3-chloro-6-méthoxyquinolin-4-yl)propyl]-1-[2-(4-fluoropyridin-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[2-(3-fluoro-pyridin-2-yl)thioéthyl]pipéridine-3-acétique
- Acide (3RS,4RS) ou (3SR,4RS)-4-[3-oxo-3-(3-chloro-6-méthoxyquinolin-4-yl)propyl]-1-[3-(3-fluoro-phényl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(2,5-difluoro-phényl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(3,5-difluoro-phényl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(2,3,5-trifluoro-phényl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(thien-2-yl)-prop-2-ynyl]-pipéridine-3-acétique
- Acide (3RS,4RS) ou (3SR,4RS)-43-oxo-3-(3-chloro-6-méthoxyquinolin-4-yl)propyl]-1-[3-(5-fluorothien-2-yl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(4-fluorothien-2-yl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(3-fluorothien-2-yl)-prop-2-ynyl]-pipéridine-3-acétique
- Acide (3RS,4RS) ou (3SR,4RS)-4-[3-oxo-3-(3-chloro-6-méthoxyquinolin-4-yl)propyl]-1-[3-(thien-3-yl)-prop-2-ynyl]-pipéridine-3-acétique

### Exemple 1

### Synthèse des 4 stéréoisomères de l'acide (3RS,4RS)-4-[3-(R,S)-hydroxy-3-(3-chloro-6-mêthoxyquinolin-4-yl)-propyl]- 1-[2-(2,5-difluoro-phénylsulfanyl)-éthyl]-pipéridine-3-carboxylique

acide (3R,4R)-4-[3-(R)-hydroxy-3-(3-chloro-6-méthoxyquinolin-4-yl)-propyl]-1-[2-(2,5-difluoro-phénylsulfanyl)-éthyl]-pipéridine-3-carboxylique
acide (3R,4R)-4-[3-(S)-hydroxy-3-(3-chloro-6-méthoxyquinolin-4-yl)-propyl]-1-[2-(2,5-difluoro-phénylsulfanyl)-éthyl]-pipéridine-3-carboxylique
acide (3S,4S)-4-[3-(S)-hydroxy-3-(3-chloro-6-méthoxyquinolin-4-yl)-propyl]-1-[2-(2,5-difluoro-phénylsulfanyl)-éthyl]-pipéridine-3-carboxylique
acide (3S,4S)-4-[3-(R)-hydroxy-3-(3-chloro-6-méthoxyquinolin-4-yl)-propyl]-1-[2-(2,5-difluoro-phénylsulfanyl)-éthyl]-pipéridine-3-carboxylique

Les quatre stéréoisomères sont nommés ci-après A, B, C, et D. Leurs stétérochimies absolues ne sont pas connues.

### Stéréoisomère A:

A 390 mg de 4-[3-hydroxy-3-(3-chloro-6-méthoxyquinolin-4-yl)-propyl]-1-[2-(2,5-difluoro-phénylsulfanyl)-éthyl]-pipéridine-3-carboxylate de méthyle (ester isomère A), dans 10 cm³ de dioxane, on ajoute 2 cm³ d'une solution aqueuse d'hydroxyde de sodium 1N. Le milieu réactionnel est ensuite chauffé à 70°C pendant 5 heures, on laisse revenir à 20 °C pendant 18 heures puis on rechauffe à 70 °C pendant 2 heures. Après retour à 20 °C, le milieu réactionnel est évaporé sous pression réduite (2 kPa ; 45 °C). Le résidu est repris dans 25 cm³ d'eau distillée et extrait avec 25 cm³ d'éther diéthylique. La phase aqueuse est acidifiée avec 1,9 cm³ d'une soluton aqueuse d'acide chlorhydrique 1N et extraite avec 3 fois 70 cm³ d'acétate d'éthyle. La phase organique est séchée sur sulfate de magnésium, filtrée sur verre fritté puis concentrée sous pression réduite (2 kPa ; 45°C). Le résidu est repris avec 25 cm³ d'acétone puis reconcentré sous pression réduite (2 kPa ; 45°C) . Après séchage à l'étuve sous pression réduite (10 kPa ; 20°C), on obtient 340 mg d'acide 4-[3-hydroxy-3-(3-chloro-6-méthoxyquinolin-4-yl)-propyl]-1-[2-(2,5-difluoro-phénylsulfanyl)-éthyl]-pipéridine-3- carboxylique (isomère A) sous forme d'un solide beige.
Spectre de R.M.N. ¹H (400 MHz, (CD₃)₂SO d6, δ en ppm) : 1,34 (mt : 1H) ; de 1,50 à 1,85 (mt : 5H) ; 2,10 (mt : 1H) ; 2,28 (mt : 1H) ; 2,43 (d très large, J = 11,5 Hz : 1H) ; de 2,45 à 2,60 (mt : 1H) ; 2,65 (t, J = 7 Hz : 2H) ; 2,73 (mf : 1H) ; 2,86 (mf : 1H) ; 3,18 (mt : 2H) ; 3,90 (s : 3H) ; 5,47 (dd, J = 9 et 5 Hz : 1H) ; 6,03 (mf : 1H) ; 7,08 (mt : 1H) ; 7,27 (mt : 1H) ; 7,34 (mt : 1H) ; 7,44 (dd, J = 9 et 3 Hz : 1H) ; 7,95 (d, J = 9 Hz : 1H) ; 8,21 (d, J = 3 Hz : 1H) ; 8,65 (s : 1H).
α_{D}²⁰= 52,3° +/- 1,1 dans le méthanol à 0,5%

### Stéréoisomère B:

A 460 mg de 4-[3-hydroxy-3-(3-chloro-6-méthoxyquinolin-4-yl)-propyl]-1-[2-(2,5-difluoro-phénylsulfanyl)-éthyl]-pipéridine-3-carboxylate de méthyle (ester isomère B),dans 10 cm³ de dioxane, on ajoute 2,4 cm³ d'une solution aqueuse d'hydroxyde de sodium 1N. Le milieu réactionnel est ensuite chauffé à 70°C pendant 5 heures, on laisse revenir à 20 °C pendant 18 heures puis on chauffe à nouveau à 70 °C pendant 2 heures. Après retour à 20 °C, le milieu réactionnel est évaporé sous pression réduite (2 kPa ; 45 °C). Le résidu est repris dans 25 cm³ d'eau distillée et extrait avec 25 cm³ de d'éther diéthylique. La phase aqueuse est acidifiée (pH=6) avec 2,3 cm³ d'une solution aqueuse d'acide chlorhydrique 1N et extraite avec 3 fois 70 cm³ d'acétate d'éthyle. La phase organique est séchée sur sulfate de magnésium, filtrée sur verre fritté puis concentrée sous pression réduite (2 kPa ; 45°C). Le résidu est repris avec 25 cm³ d'acétone puis reconcentrée sous pression réduite (2 kPa ; 45°C). Après séchage à l'étuve sous pression réduite (10 kPa ; 20°C), on obtient 310 mg d' acide 4-[3-hydroxy-3-(3-chloro-6-méthoxyquinolin-4-yl)-propyl]-1-[2-(2,5-difluorophénylsulfanyl)-éthyl]-pipéridine-3-carboxylique(isomère B) sous forme d'un solide jaune pâle.
Spectre de R.M.N. ¹H (400 MHz, (CD₃)₂SO d6 à une température de 303K, δ en ppm) : de 1,20 à 1,40 (mt : 1H) ; de 1,50 à 1,85 (mt : 5H) ; de 2,00 à 2,15 (mt : 1H) ; de 2,20 à 2,55 (mf étalé : 2H) ; 2,60 (mt : 1H) ; de 2,60 à 3,05 (mt : 4H) ; 3,22 (mt : 2H) ; 3,90 (s : 3H) ; 5,46 (mt : 1H) ; 6,01 (d, J = 3,5 Hz : 1H) ; 7, 10 (mt : 1H) ; 7,29 (mt : 1H) ; 7,36 (mt : 1H) ; 7,44 (dd, J = 9 et 3 Hz : 1H) ; 7,95 (d, J = 9 Hz : 1H) ; 8,21 (d, J = 3 Hz : 2H) ; 8,65 (s : 1H) .
α_{D}²⁰=-53, 1° +/- 1,1 dans le méthanol à 0,5%

### Stéréoisomère C:

A 270 mg de 4-[3-hydroxy-3-(3-chloro-6-méthoxyquinolin-4-yl)-propyl]-1-[2-(2,5-difluoro-phénylsulfanyl)-éthyl]-pipéridine-3-carboxylate de méthyle (ester isomère C),dans 10 cm³ de dioxane, on ajoute 1,4 cm³ d'une solution aqueuse d'hydroxyde de sodium 1N. Le milieu réactionnel est ensuite chauffé à 70°C pendant 5 heures, on laisse revenir à 20 °C pendant 18 heures puis on chauffe à nouveau à 70 °C pendant 4 heures. Après retour à 20 °C, le milieu réactionnel est évaporé sous pression réduite (2 kPa ; 45 °C). Le résidu est repris dans 25 cm³ d'eau distillée et extrait avec 25 cm³ de d'éther diéthylique. La phase aqueuse est acidifiée (pH=6) avec 1,3 cm³ d'une solution aqueuse d'acide chlorhydrique 1N et extraite avec 3 fois 70 cm³ d'acétate d'éthyle. La phase organique est séchée sur sulfate de magnésium , filtrée sur verre fritté puis concentrée sous pression réduite (2 kPa ; 45°C). Le résidu est repris avec 25 cm³ d'acétone puis reconcentrée sous pression réduite (2 kPa ; 45°C). Après séchage à l'étuve sous pression réduite (10 kPa ; 20°C), on obtient 310 mg d' acide 4-[3-hydroxy-3-(3-chloro-6-méthoxyquinolin-4-yl)-propyl]- 1-[2-(2,5-difluorophénylsulfanyl)-éthyl]-pipéridine-3-carboxylique(isomère C) sous forme d'un solide beige.
Spectre de R.M.N. ¹H (300 MHz, (CD₃)₂SO d6, δ en ppm) : de 1,20 à 1,40 (mt : 1H) ; de 1,45 à 1,90 (mt : 5H) ; de 2,05 à 2,30 (mt : 2H) ; 2,39 (d très large, J = 10,5 Hz : 1H) ; 2,56 (mt : 1H) ; 2,64 (t, J = 7 Hz : 2H) ; de 2,65 à 2,80 (mf : 1H) ; 2,92 (mt : 1H) ; 3,17 (mt : 2H) ; 3,90 (s : 3H) ; 5,45 (dd, J = 8,5 et 5 Hz : 1H) ; 6,01 (mf : 1H) ; 7,08 (mt : 1H) ; de 7,20 à 7,40 (mt : 2H) ; 7,43 (dd, J = 9 et 3 Hz : 1H) ; 7,94 (d, J = 9 Hz : 1H) ; 8,22 (d, J = 3 Hz : 1H) ; 8,64 (s : 1H).
α_{D}²⁰ = 60,1° +/- 1,2 dans le méthanol à 0,5%

### Stéréoisomère D:

A 270 mg de 4-[3- hydroxy-3-(3-chloro-6-méthoxyquinolin-4-yl)-propyl]-1-[2-(2,5-difluoro-phénylsulfanyl)-éthyl]-pipéridine-3-carboxylate de méthyle (ester isomère D),dans 10 cm³ de dioxane, on ajoute 1,4 cm³ d'une solution aqueuse d'hydroxyde de sodium 1N. Le milieu réactionnel est ensuite chauffé à 70°C pendant 5 heures, on laisse revenir à 20 °C pendant 18 heures puis on chauffe à nouveau à 70 °C pendant 4 heures. Après retour à 20 °C, le milieu réactionnel est évaporé sous pression réduite (2 kPa ; 45 °C). Le résidu est repris dans 25 cm³ d'eau distillée et extrait avec 25 cm³ de d'éther diéthylique. La phase aqueuse est acidifiée (pH=6) avec 1,3 cm³ d'une solution aqueuse d'acide chlorhydrique 1N et extraite avec 3 fois 70 cm³ d'acétate d'éthyle. La phase organique est séchée sur sulfate de magnésium , filtrée sur verre fritté puis concentrée sous pression réduite (2 kPa ; 45°C). Le résidu est repris avec 25 cm³ d'acétone puis reconcentrée sous pression réduite (2 kPa ; 45°C). Après séchage à l'étuve sous pression réduite (10 kPa ; 20°C), on obtient 200 mg d' acide 4-[3-hydroxy-3-(3-chloro-6-méthoxyquinolin-4-yl)-propyl]- 1-[2-(2,5-difluorophénylsulfanyl)-éthyl]-pipéridine-3-carboxylique (isomère D) sous forme d'un solide blanc.
Spectre de R.M.N. ¹H (300 MHz, (CD₃)₂SO d6, δ en ppm) : de 1,20 à 1,40 (mt : 1H) ; de 1,45 à 1,85 (mt : 5H) ; de 2,05 à 2,30 (mt : 2H) ; 2,39 (de très large, J = 10, 5 Hz : 1H) ; 2,56 (mt : 1H) ; de 2,60 à 2,80 (mf : 1H) ; 2,64 (t, J = 7 Hz : 2H) ; 2,91 (mt : 1H) ; 3,17 (mt : 2H) ; 3,90 (s : 3H) ; 5,45 (dd, J = 8,5 et 5 Hz : 1H) ; 6,01 (mf : 1H) ; 7,08 (mt : 1H) ; de 7,20 à 7,40 (mt : 2H) ; 7,43 (dd, J = 9 et 3 Hz : 1H) ; 7,94 (d, J = 9 Hz : 1H) ; 8,22 (d, J = 3 Hz : 1H) ; 8,64 (s : 1H).
α_{D}²⁰= -60,1° +/- 1,2 dans le méthanol à 0,5%
(3RS,4RS)-4-[3-(R,S)-hydroxy-3-(3-chloro-6-méthoxyquinolin-4-yl)-propyl]-2-[2-(2,5-difluoro-phénylsulfanyl)-éthyl]- pipéridine-3-carboxylate de méthyle
(3R,4R)-4-[3-(R)-hydroxy-3-(3-chloro-6-méthoxyquinolin-4-yl)-propyl]-1-[2-(2,5-difluoro-phénylsulfanyl)-éthyl]-pipéridine-3-carboxylate de méthyle
(3S,4S)-4-[3-(S)-hydroxy-3-(3-chloro-6-méthoxyquinolin-4-yl)-propyl]-1-[2-(2,5-difluoro-phénylsulfanyl)-éthyl]-pipéridine-3-carboxylate de méthyle
(3S,4S)-4-[3-(R,)-hydroxy-3-(3-chloro-6-méthoxyquinolin-4-yl)-propyl]-1-[2-(2,5-difluoro-phénylsulfanyl)-éthyl]-pipéridine-3-carboxylate de méthyle
(3S,4S)-4-[3-(S,)-hydroxy-3-(3-chloro-6-méthoxyquinolin-4-yl)-propyl]-1-[2-(2,5-difluoro-phénylsulfanyl)-éthyl]-pipéridine-3-carboxylate de méthyle

Les quatre stéréoisomères sont nommés ci-après A,B,C, et D. Leurs stéréochimies absolues ne sont pas connues.

A 2,35 g de chlorhydrate de (3RS,4RS)-4-[3-(R,S)-hydroxy-(3-chloro-6-méthoxyquinolin-4-yl)propyl]-pipéridine-3-carboxylate de méthyle chlohydrate solubilisé dans 110 cm³ d'acétonitrile, on ajoute 1,5 cm³ de triéthylamine, 2,15 g de carbonate de potassium et 0.85 g de iodure de potassium. On ajoute ensuite 1,3 g de 1-(2-bromoéthylsulfanyl)-(2,5-difluoro)-benzène. Le milieu réactionnel est ensuite porté à 60 °C pendant 16 heures. On laisse ensuite revenir le milieu à 20 °C, il est ensuite filtré sur verre fritté n° 3 puis on lave avec 2 fois 20 cm³ d'acétonitrile puis on évapore sous pression réduite (45 °C ; 5 kPa). Le résidu est purifié par chromatographie, sous une pression d'argon de 150 kPa, sur une colonne de gel de silice (granulométrie 0,065-0,2 µm ; diamètre 2,5 cm ; hauteur 40 cm), en éluant par un mélange de cyclohexane-acétate d'éthyle (60/40 en volumes) et en recueillant des fractions de 50 cm³. Les fractions 8 à 16 sont réunies, puis concentrées sous pression réduite (45 °C ; 5 kPa). On obtient 2,15 g de (3RS,4RS)-4-[3-(R,S)-hydroxy-3-(3-chloro-6-méthoxyquinolin-4-yl)-propyl]-1-[2-(2,5-difluorophénylsulfanyl)-éthyl]-pipéridine-3-carboxylate de méthyle (mélange des isomères A,B,C,D) sous forme d'une huile incolore.
Le 1-(2-bromoéthylsulfanyl)-(2,5-difluoro)-benzène est préparé selon la demande de brevet W0200240474.
Spectre de R.M.N. ¹H (300 MHz, (CD₃)₂SO d6, δ en ppm). Nous observons un mélange de diastéréoisomère dans les proportions 50/50.
* de 1,10 à 1,90 (mt : 6H) ; de 1,90 à 2,90 (mt : 7H) ; 2,37 (d large, J = 10, 5 Hz : 1H) ; 3,10 (t, J = 7 Hz : 2H) ; 3,40 et 3,55 (2s : 3H en totalité) ; 3,88 et 3,89 (2s : 3H en totalité) ; 5,44 (mt : 1H) ; 6,01 (s large : 1H) ; 7,05 (mt : 1H) ; de 7,20 à 7,35 (mt : 2H) ; 7,44 (dd, J = 9 et 3 Hz : 1H) ; 7,95 (d, J = 9 Hz : 1H) ; 8,19 (mt : 1H) ; 8,65 et 8,66 (2s : 1H en totalité).

A partir du mélange de stéréoisomères A,B,C,D obtenus précédemment, la séparation de chaque stéréoisomère s'effectue par HPLC.

La séparation des 2 couples de stéréoisomères (A+B) et (C+D), est réalisée sur une phase stationnaire Simmetry C18 à partir de 2,7 g du mélange A, B, C, D décrit précédemment, granulométrie 7 µmm ; diamètre 60 mm ; masse de la phase stationnaire 700 g), sous une pression de 500 kPa, la phase mobile est composée d'un mélange de méthanol-solution tampon aqueuse (pH=4.9)-acétonitrile (10/30/60 en volumes) ayant un débit de 120 cm³ par minute et la longueur d'onde du détecteur UV est fixée à 280 nm.
Les fractions contenant la première paire d'énantiomères notée (A+B) sont réunies et évaporées sous pression réduite (2 kPa) à une température voisine de 40°C . Le résidu obtenu est repris dans de l'eau puis extrait 2 fois avec du dichlorométhane. La phase organique est séchée sur sulfate de magnésium, filtrée puis évaporée sous pression réduite (2 kPa ; 45 °C). On obtient 850 mg de produit (mélange A+B).
Les fractions contenant la deuxième paire d'énantiomères notée (C+D) sont réunies et évaporées sous pression réduite (2 kPa) à une température voisine de 40°C. Le résidu obtenu est repris dans de l'eau puis extrait avec 2 fois du dichlorométhane. La phase organique est séchée sur sulfate de magnésium, filtrée puis évaporée sous pression réduite (2 kPa ; 45 °C).On obtient 540 mg de produit (mélange C+D).

Ensuite les produits du couple d'énantiomères (A,B) sont séparés sur une colonne chiralcel OD (granulométrie 20 µmm ; diamètre 80 mm ; masse de la phase stationnaire 1250 g) sous une pression de 1000 kPa, la phase mobile est composée d'un mélange de heptane-isopropanol-méthanol-triéthylamine (90/5/5/0.1 en volumes) ayant un débit de 150 cm³ par minute et la longueur d'onde du détecteur UV est fixée à 280 nm. Les fractions contenant chaque produit sont isolées puis concentrées sous une pression réduite (3 kPa) à une température voisine de 40°C ; on obtient 0,391 g de l'énantiomère A, et 0,459 g de l'énantiomère B.

De même, les produits du couple d'énantiomères (C,D) sont séparés sur une colonne chiralpak AD (granulométrie 20 µmm ; diamètre 80 mm ; masse de la phase stationnaire 750 g) sous une pression de 1000 kPa, la phase mobile est composée d'un mélange de heptane-isopropanol-méthanol-triéthylamine (80/10/10/0.1 en volumes) ayant un débit de 100 cm³ par minute et la longueur d'onde du détecteur UV est fixée à 280 nm. Les fractions contenant chaque produit sont isolées puis concentrées sous une pression réduite (3 kPa) à une température voisine de 40°C ; on obtient 0,27 g de l'énantiomère C et 0,27 g de l'énantiomère D.

### Stéréoisomère A

Spectre de R.M.N. ¹H (300 MHz, (CD₃)₂SO d6, δ en ppm) : de 1,10 à 1,30 (mt : 1H) ; 1,50 (mt : 1H) ; de 1,60 à 1,85 (mt : 4H) ; 2,08 (mt : 1H) ; 2,22 (mt : 1H) ; 2,36 (d très large, J = 10,5 Hz : 1H) ; de 2,45 à 2,60 (mt : 3H) ; 2,63 (mt : 1H) ; 2,75 (mt : 1H) ; 3,10 (t, J = 7 Hz : 2H) ; 3,40 (s : 3H) ; 3,88 (s : 3H) ; 5,44 (mt : 1H) ; 6,02 (d, J = 3,5 Hz : 1H) ; 7,05 (mt : 1H) ; de 7,20 à 7,35 (mt : 2H) ; 7,43 (dd, J = 9 et 3 Hz : 1H) ; 7,95 (d, J = 9 Hz : 1H) ; 8,19 (d, J = 3 Hz : 1H) ; 8,65 (s : 1H).
α_{D}²⁰= 40,2° +/- 0,9 dans le DMSO à 0,5%
Condition HPLC : colonne Chiralcel OD, débit 1 cm³/min, condition d'élution
de 0 à 16 min : heptane-isopropanol-éthanol-triéthylamine (88/6/6/0,1 en volumes)
Temps de rétention : 10,47 min

### Stéréoisomère B

Spectre de R.M.N. ¹H (300 MHz, (CD₃)₂SO d6, δ en ppm) : de 1,10 à 1,30 (mt : 1H) ; 1,51 (mt : 1H) ; de 1,60 à 1,85 (mt : 4H) ; de 2,00 à 2,20 (mt : 1H) ; 2,23 (mt : 1H) ; 2,37 (d très large, J = 20,5 Hz : 1H) ; de 2,45 à 2,60 (mt : 3H) ; 2,64 (mt : 1H) ; 2,75 (mt : 1H) ; 3,11 (t, J = 7 Hz : 2H) ; 3,41 (s : 3H) ; 3,89 (s : 3H) ; 5,45 (mt : 1H) ; 6,03 (d, J = 4 Hz : 1H) ; 7,07 (mt : 1H) ; de 7,20 à 7,35 (mt : 2H) ; 7,44 (dd, J = 9 et 3 Hz : 1H) ; 7,95 (d, J = 9 Hz : 1H) ; 8,20 (d, J = 3 Hz : 1H) ; 8,66 (s : 1H) .
α_{D}²⁰= -38,3° +/- 0,9 dans le DMSO à 0,5%
Condition HPLC : colonne Chiralcel OD, débit 1 cm³/min, condition d'élution
de 0 à 16 min : heptane-isopropanol-éthanol-triéthylamine (88/6/6/0,1 en volumes)
Temps de rétention : 13,95 min

### Stéréoisomère C

Spectre de R.M.N. ¹H (300 MHz, (CD₃)₂SO d6, δ en ppm) : de 1,30 à 1,55 (mt : 2H) ; de 1, 55 à 1,90 (mt : 4H) ; 1,97 (mt : 1H) ; 2,19 (mt : 1H) ; 2,37 (d très large, J = 10,5 Hz : 1H) ; de 2,40 à 2,65 (mt : 3H) ; 2,68 (mt : 1H) ; 2,80 (mt : 2H) ; 3, 11 (t, J = 7 Hz : 2H) ; 3,55 (s : 3H) ; 3,90 (s : 3H) ; 5, 45 (mt : 1H) ; 6,03 (d, J = 3,5 Hz : 1H) ; 7,06 (mt : 1H) ; de 7,20 à 7,35 (mt : 2H) ; 7,44 (dd, J = 9 et 3 Hz : 1H) ; 7,96 (d, J = 9 Hz : 1H) ; 8,20 (d, J = 3 Hz : 1H) ; 8,66 (s : 1H).
□_{D}²⁰= 26,6° +/- 0,8 dans le DMSO à 0,5%
Condition HPLC : colonne Chiralpak AD, débit 1 cm³/min, condition d'élut ion
de 0 à 20 min : heptane-isopropanol-éthanol-triéthylamine (88/5/7/0,1 en volumes)
Temps de rétention : 13,01 min

### Stéréoisomère D

Spectre de R.M.N. ¹H (300 MHz, (CD₃)₂SO d6, δ en ppm) : de 1,30 à 1,55 (mt : 2H) ; de 1,55 à 1,85 (mt : 4H) ; 1,97 (mt : 1H) ; 2,18 (mt : 1H) ; 2,37 (d très large, J = 10,5 Hz : 1H) ; de 2,40 à 2,65 (mt : 3H) ; 2,69 (mt : 1H) ; 2,79 (mt : 1H) ; 3,11 (t, J = 7 Hz : 2H) ; 3,55 (s : 3H) ; 3,89 (s : 3H) : 5,45 (mt : 1H) ; 6,03 (d, J = 3,5 Hz : 1H) ; 7,06 (mt : 1H) ; de 7,20 à 7,35 (mt : 2H) ; 7,44 (dd, J = 9 et 3 Hz : 1H) ; 7, 96 (d, J = 9 Hz : 1H) ; 8,21 (d, J = 3 Hz : 1H) ; 8,66 (s : 1H).
α_{D}²⁰= - 27,4° +/- 0,8 dans le DMSO à 0,5%
Condition HPLC : colonne Chiralpak AD, débit 1 cm³/min, condition d'élution
de 0 à 20 min : heptane-isopropanol-éthanol-triéthylamine (88/5/7/0,1 en volumes)
Temps de rétention : 15,21 min

### Chlorhydrate de (3RS, 4RS)-4-[3-(RS)-hydroxy-3-(3-chloro-6-méthoxyquinolin-4-yl)propyl]-1-H-pipéridine-3-carboxylate de méthyle

A 5,08 g d'acide (3RS, 4RS)-4-[3-(R,S)-hydroxy-(3-chloro-6-méthoxyquinolin-4-yl)propyl]-1-(tert-butyloxycarbonyl)-pipéridine-3-carboxylique dans 110 cm³ de méthanol, on ajoute 3,1 cm³ de chlorure de thionyle après avoir refroidit vers -25 °C à l'aide d'un bain d'acétone et carboglace, goutte à goutte en 45 minutes, puis on laisse revenir à 20 °C pendant 16 heures. Le milieu réactionnel est ensuite évaporé sous pression réduite (45 °C ; 5 kPa). Le résidu est repris avec 100 cm³ d'éther isopropylique et trituré jusqu'à obtenir une poudre fine. On concentre ensuite sous pression réduite (45 °C ; 5 kPa). Le produit obtenu est solubilisé dans 100 cm³ de méthanol. On ajoute encore 3,4 cm³ de chlorure de thionyle après avoir refroidi vers -20°C. On laisse, à nouveau agiter pendant 16 heures puis on concentre à sec sous pression réduite (45°C ; 5 kPa). Le résidu est repris avec 60 cm³ d'éther isopropylique , concentré à sec sous pression réduite (45°C ; 5 kPa). On obtient 4,75 g de (3RS, 4RS)-4-[3-(RS)-hydroxy-3-(3-chloro-6-méthoxyquinolin-4-yl)propyl]-1-H-pipéridine-3-carboxylate de méthyle sous forme de chlorhydrate, solide beige.
Spectre de R.M.N. ¹H (300 MHz, (CD₃)₂SO d6, δ en ppm) : On observe un mélange de 2 diastéréoisomères dans les proportions 60/40.
de 1,05 à 2,20 (mt : 8H) ; de 2,80 à 3,35 (mt : 4H) ; 3,46 et 3,65 (2 s : 3H en totalité) ; 3,92 et 3,93 (2 s : 3H en totalité) ; 5,48 (mt : 1H) ; 7,47 (dd, J = 9 et 3 Hz : 1H) ; 7,98 (d, J = 9 Hz : 1H) ; de 8,10 à 8,30 (mf : 1H) ; 8,23 (mt : 1H) ; 8,69 (s : 1H) ; de 9,00 à 9,35 (mf étalé : 1H en totalité).
IC: m/z 393 (M+H)⁺

### Acide (3RS,4RS)-4-[3-(R,S)-hydroxy-3-(3-chloro-6-méthoxyquinolin-4-yl)propyl]-1-(tert-butyloxycarbonyl)-pipéridine-3-carboxylique.

A 5,55 g d'acide (3RS, 4RS)-4-[3-(3-chloro-6-méthoxyquinolin-4-yl)propyl]-1-(tert-butyloxycarbonyl)-pipéridine-3-carboxylique dans 450 cm³ de diméthylsulfoxyde, on ajoute 100 cm³ de ter-butanol puis on sature le milieu réactionnel en oxygène pendant 30 minutes. On ajoute ensuite en 40 minutes une solution de 3,36 g de tert-butoxyde de potassium dans 40 cm³ de ter-butanol. On laisse agiter pendant 2 heures en maintenant le débit d'oxygène puis on refroidit le milieu vers 0 °C pour ajouter 1,8 cm³ d'acide acétique dans 30 cm³ d'eau distillée. On ajoute ensuite sur le milieu réactionnel, 1000 cm³ d'eau distillée et 1000 cm³ d'acétate de méthyle. La phase organique est ensuite lavée avec 8 fois 250 cm³ d'eau distillée puis avec 2 fois 100 cm³ de chlorure de sodium. Les phases aqueuses réunies sont réextraites avec 500 cm³ d'acétate d'éthyle. Les deux phases organiques sont réunies puis séchées sur sulfate de magnésium pendant 1 heure. On filtre sur verre fritté puis on concentre sous pression réduite (2 kPa ; 45 °C). Le résidu est repris dans 250 cm³ d'acétate d'éthyle et 100 cm³ d'eau distillée. On lave la phase organique avec 3 fois 50 cm³ d'eau distillée puis avec 50 cm³ d'une solution aqueuse saturée en chlorure de sodium. On sèche sur sulfate de magnésium pendant 1 heure, on filtre sur verre fritté puis on évapore sous pression réduite (2 kPa ; 45 °C). On obtient 5,08 g d'acide (3RS, 4RS)-4-[3-(R,S)-hydroxy-3-(3-chloro-6-méthoxyquinolin-4-yl)propyl]-1-(tert-butyloxycarbonyl)-pipéridine-3-carboxylique.

Spectre de R.M.N. ¹H (300 MHz, (CD₃)₂SO d6, δ en ppm). On observe un mélange de diastéréoisomères
* de 1,20 à 1,90 (mt : 6H) ; 1,38 (s large : 9H) ; de 2,00 à 2,20 (mt : 1H) ; 2,45 (mt : 1H) ; de 2,65 à 4,00 (mf étalé : 4H) ; 3,89 (s : 3H) ; 5,46 (mt : 1H) ; de 5,90 à 6,15 (mf étalé : 1H) ; 7,43 (dd, J = 9 et 3 Hz : 1H) ; 7,95 (d, J = 9 Hz : 1H) ; 8,20 (mt : 1H) ; 8,64 et 8,65 (2s : 1H en totalité) ; de 12,70 à 12,20 (mf étalé : 1H).

EI m/z=478 M⁺
m/z=405 [M - OtBu]⁺
m/z=377 [M - BOC]⁺
m/z=223 [C₁₁H₁₀O₂NCl]^{+.}
m/z=194 [223 - CHO]⁺
m/z=57 [C₄H₉]⁺ pic de base

DCI m/z=479 MH⁺

### Acide (3RS, 4RS)-4-[3-(3-chloro-6-méthoxyquinolin-4-yl)propyl]-1-(tert-butyloxycarbonyl)-pipéridine-3-carboxylique.

A 7,05 g de (3RS, 4RS)-4-[3-(3-chloro-6-méthoxyquinolin-4-yl)propyl]-1-(tert butyloxycarbonyl)-pipéridine-3-carboxylate de méthyle, dans 100 cm³ de dioxane, on additionne 60 cm³ d'une solution aqueuse d'hydroxyde de sodium 1N. Le milieu réactionnel est ensuite chauffé à 60°C pendant 2 heures puis concentré à sec sous pression réduite (45°C ; 5 kPa). Le résidu obtenu est repris avec 300 cm³ d'éther diéthylique et 500 cm³ d'eau distillée. La phase aqueuse est ensuite lavée avec 200 cm³ d'éther diéthylique puis acidifiée avec 55 cm³ d'une solution aqueuse d'acide chlorhydrique 1N. On réextrait ensuite avec 2 fois 250 cm³ d'acétate d'éthyle. Les phases organiques réunies sont séchées sur sulfate de magnésium pendant 1 heure puis on filtre sur verre fritté et on évapore sous pression réduite (45°C ; 5 kPa). On obtient 5,5 g d' acide (3RS, 4RS)-4-[3-(3-chloro-6-méthoxyquinolin-4-yl)propyl]-1-(tert-butyloxycarbonyl)-pipéridine-3-carboxylique sous forme d'un solide blanc
Spectre de R.M.N. ¹H (400 MHz, (CD₃)₂SO d6, δ en ppm) : de 1,35 à 1,95 (mt : 7H) ; 1,39 (s : 9H) ; de 2,45 à 2,60 (mt : 1H) ; de 2,85 à 4,00 (mf étalé : 4H) ; 3,20 (t large, J = 6 Hz : 2H) ; 3,97 (s large : 3H) ; 7,38 (d, J = 3 Hz : 1H) ; 7,45 (dd, J = 9 et 3 Hz : 1H) ; 7,96 (d, J = 9 Hz : 1H) ; 8,67 (s : 1H) ; de 11, 90 à 12,50 (mf très étalé : 1H) .
IC: m/z 463 (M+H)⁺

### (3RS,4RS)-4-[3-(3-chloro-6-méthoxyquinolin-4-yl)propyl]-1-(tert butyloxycarbonyl)-pipéridine-3-carboxylate de méthyle

A 72 cm³ d'une solution 0,5 M de 9-borabicyclo[3,3,1]nonane dans le tétrahydrofurane sous agitation et sous atmosphère inerte et après avoir refroidi à 0 °C, on ajoute 5,85 g de (3RS, 4RS)-1-(tert-butyloxycarbonyl)-4-allyl-pipéridine-3-carboxylate de méthyle (isomère A), solubilisé dans 60 cm³ de tétrahydrofurane. Le mélange est ensuite ramené à une température voisine de 20°C, tandis que l'agitation est poursuivie pendant encore 4 heures. 6,03 g de 4-bromo-3-chloro-6-méthoxy quinoléine en solution dans 200 cm³ de tétrahydrofurane sont ajoutés en 45 minutes, puis 440 mg de chlorure de palladium diphénylphosphinoferrocène et enfin 12.8 g de phosphate de potassium tribasique. Le mélange réactionnel est chauffé pendant 15 heures au reflux puis filtré à chaud sur verre fritté. Le filtrat est repris dans 4 fois 20 cm³ d'acétate d'éthyle et concentré à sec sous pression réduite (45 °C ; 5kPa). Le résidu est repris par 250 cm³ d'acétate d'éthyle et 200 cm³ d'eau. La phase organique est décantée, lavée avec 3 fois 50 cm³ d'eau distillée et avec 2 fois 100 cm³ d'une solution aqueuse saturée de chlorure de sodium, séchée sur sulfate de magnésium, filtrée, puis concentrée sous pression réduite (45 °C ; 5 kPa). Le résidu est purifié par chromatographie, sous une pression d'argon de 150 kPa, sur une colonne de gel de silice (granulométrie 20-45µ ; diamètre 8 cm ; hauteur 35 cm), en éluant par un mélange de cyclohexane-acétate-d'éthyle (73/27 en volumes) et en recueillant des fractions de 200 cm³. Les fractions 8 à 16 sont réunies, puis concentrées sous pression réduites (45 °C ; 5 kPa). On obtient 9,5 g de (3RS,4RS)-4-[3-(3-chloro-6-méthoxyquinolin-4-yl)-3-propyl]-1-(tert-butyloxycarbonyl)-pipéridine-3-carboxylate de méthyle sous forme d'une huile incolore.
Spectre de R.M.N. ¹H (300 MHz, (CD₃)₂SO d6, δ en ppm) : de - 1,30 à 1,90 (mt : 7H) ; 1,37 (s : 9H) ; 2,63 (mt : 1H) ; de 2,70 à 3,25 (mf : 2H) ; 3,18 (t large, J = 7,5 Hz : 2H) ; 3,51 (s large : 3H) ; de 3,60 à 4,00 (mf : 2H) ; 3,97 (s : 3H) ; 7,38 (d, J = 3 Hz : 1H) ; 7,45 (dd, J = 9 et 3 Hz : 1H) ; 7, 96 (d, J = 9 Hz : 1H) ; 8,67 (s : 1H).
IE: m/z 476 (M⁺), m/z 375, 207,194,170, 58 (pic de base)

La 4-bromo-3-chloro-6-méthoxy quinoléine est décrite dans la demande de brevet WO20024074.

### Synthèses des 2 couples de stéréoisomères de 1-(tert-butyloxycarbonyl)-4-allyl-pipéridine-3-carboxylate de méthyle.

(3RS, 4RS)-1-(tert-butyloxycarbonyl)-4-allyl-pipéridine-3-carboxylate de méthyle (isomère A, racémique)
(3RS,4SR)-1-(tert-butyloxycarbonyl)-4-allyl-pipéridine-3-carboxylate de méthyle(isomère B, racémique)

Une solution de 32,43 g de 1-(tert-butyloxycarbonyl)-4-allyl-4-(méthoxyoxalyloxy)-hydroxy-pipéridine-3-carboxylate de méthyle (racémique A) dans 600 cm³ de toluène sous atmosphère inerte est chauffée à une température de 110°. On ajoute ensuite rapidement 200 mg d'AIBN puis 35,06 cm³ d'hydrure de tributylétain puis à nouveau 200 mg d'AIBN. Le milieu est maintenu à 110°C pendant 4 heures. Le mélange est ensuite refroidi à une température proche de 20°C pendant 12 heures, puis on ajoute 300 cm³ d'eau distillée. La phase organique est relavée avec 3 fois 300 cm³ d'eau distillée puis séchée sur sulfate de magnésium filtrée sur verre fritté et, concentrée à sec sous pression réduite (45°C; 5kPa). Le résidu est purifié par chromatographie, sous une pression d'azote de 50 kPa, sur une colonne de gel de silice (granulométrie 0,06-0,2mm ; diamètre 12 cm ; hauteur 75 cm), en éluant par un mélange de cyclohexane-acétate d'éthyle (80/20 en volumes) et en recueillant des fractions de 100 cm³ . Les fractions 45 à 103 sont réunies, puis concentrées. On obtient 16,05 g d'un mélange d'isomères (A+B) de 1-(tert-butyloxycarbonyl)-4-allyl-pipéridine-3-carboxylate de méthyle sous forme d'une huile jaune claire .
Spectre de R.M.N. ¹H (300 MHz, (CD₃)₂SO d6, δ en ppm) : 1,38 (s : 9H) ; 1,43 (mt : 1H) ; 1,75 (mt : 1H) ; 1,66 (mt : 1H) ; 2,06 (mt : 2H) ; 2,61 (q, J = 5,5 Hz : 1H) ; de 2,75 à 3,15 (mf étalé : 1H) ; 3,20 (dd, J = 13,5 et 5,5 Hz : 1H) ; 3,59 (s large : 3H) ; de 3,60 à 4,10 (mf étalé : 2H) ; 5,01 (mt : 2H) ; 5,75 (mt : 1H) .
IC: m/z 284 (M+H)⁺

A partir du mélange d'isomères (A+B) obtenus précédemment, la séparation des 2 couples d'isomères s'effectue par HPLC.

La séparation de A (racémique) et B (racémique) est réalisée sur une phase stationnaire Kromasil C8 à partir de 16,08 g du mélange A+B décrit précédemment, (granulométrie 10 µmm ; diamètre 80 mm ; masse de la phase stationnaire 1,25 kg), sous une pression de 600 kPa, la phase mobile est composée d'un mélange de acétone-eau distillée (60/40 en volumes) ayant un débit de 126 cm³ par minute et la longueur d'onde du détecteur UV est fixée à 215 nm. Les fractions contenant le premier isomère noté A (racémique) sont réunies et évaporées sous pression réduite (2 kPa) à une température voisine de 40°C. On obtient 6,55 g de (3RS, 4RS)-1-(tert-butyloxycarbonyl)-4-allyl-pipéridine-3-carboxylate de méthyle. Les fractions contenant le deuxième isomère noté B (racémique) sont réunies et évaporées sous pression réduite (2 kPa) à une température voisine de 40°C. On obtient 2,35 g de (3RS, 4SR)-1-(tert-butyloxycarbonyl)-4-allyl-pipéridine-3-carboxylate de méthyle.

### Isomère A (racémique)

Spectre de R.M.N. ¹H (300 MHz, (CD₃)₂SO d6, δ en ppm) : 1,38 (s : 9H) ; 1,43 (mt : 1H) ; 1,75 (mt : 1H) ; 1,66 (mt : 1H) ; 2,06 (mt : 2H) ; 2,61 (q, J = 5,5 Hz : 1H) ;de 2,75 à 3,15 (mf étalé : 1H) ; 3,20 (dd, J = 13,5 et 5,5 Hz : 1H) ; 3,59 (s large : 3H) ; de 3,60 à 4,10 (mf étalé : 2H) ; 5,01 (mt : 2H) ; 5,75 (mt : 1H).
IC: m/z 284 (M+H)⁺
Condition HPLC : colonne préparative, Kromasil C8, débit 1 cm³/min, condition d'élution
de 0 à 16 min : acétonitrile-eau distillée (60/40)
Temps de rétention : 13,18 min

### Isomère B (racémique)

Spectre de R.M.N. ¹H (400 MHz, (CD₃)₂SO d6, δ en ppm), à une température de 373K : 1,13 (mt : 1H) ; 1,43 (s : 9H) ;1,73 (dq, J = 14 et 4 Hz : 1H) ; 1,87 (mt : 1H) ; 1,97 (mt : 1H) ; 2,15 (mt : 1H) ; 2,21 (t dédoublé, J = 10 et 4 Hz : 1H) ; 2,83 (ddd, J = 13,5 - 12 et 3 Hz : 1H) ; 2,89 (dd, J = 13 et 11 Hz : 1H) ; 3,67 (s : 3H) ; 3,89 (d mt, J = 13,5 Hz : 1H) ; 4,02 (ddd, J = 13 - 4 et 2 Hz : 1H) ; 5,04 (mt : 2H) ; 5,76 (mt : 1H).
IC: m/z 284 (M+H)⁺
Condition HPLC : colonne préparative, Kromasil C8, débit 1 cm³/min, condition d'élution
de 0 à 16 min : acétonitrile-eau distillée (60/40)
Temps de rétention : 11,37 min

### 1-(tert-butyloxycarbonyl)-4-allyl-4-(méthoxyoxalyloxy)-hydroxy-pipéridine-3-carboxylate de méthyle

On ajoute, sous atmosphère inerte, 45,5 g de diméthylaminopyridine à une solution de 36,8 g de 1-(tert-butyloxycarbonyl)-4-allyl-4-hydroxy-pipéridine-3-carboxylate de méthyle dans 400 cm³ d'acétonitrile puis on additionne en 30 minutes 35,32 cm³ de chlorure d'oxalyle. Après 20 heures d'agitation à une température proche de 20°C. Le milieu réactionnel est repris par 300 cm³ d'acétate d'éthyle et 500 cm³ d'une solution aqueuse saturée de bicarbonate de sodium. La phase organique est décantée, lavée avec 6 fois 300 cm³ d'eau distillée puis avec 2 fois 300 cm³ d'une solution aqueuse saturée de chlorure de sodium. De même, la phase aqueuse est lavée avec 3 fois 300 cm³ d'acétate de méthyle. Les phases organiques regroupées sont séchées sur sulfate de magnésium, filtrées sur verre fritté. Le résidu est purifié par chromatographie, sous une pression d'azote de 50 kPa, sur une colonne de gel de silice (granulométrie 40-60 µm ; diamètre 8 cm ; hauteur 60 cm), en éluant par un mélange de cyclohexane-acétate d'éthyle (70/30 en volumes) et en recueillant des fractions de 250 cm³ . Les fractions 19 à 37 sont réunies, puis concentrées sous pression réduite. On obtient 23,31 g d'un mélange d'isomères (A+B) de 1-(tert-butyloxycarbonyl)-4-allyl-4-(méthoxyoxalyloxy)-hydroxypipéridine-3-carboxylate de méthyle sous forme d'une huile jaune claire.
Spectre de R.M.N. ¹H (300 MHz, (CD₃)₂SO d6, δ en ppm) . On observe un mélange de diastéréoisomères dans les proportions 65/35.

* 1,39 et 1,42 (2 s : 9H en totalité) ; 1,85 - 2,17 et 2,32 (3 mts : 2H en totalité) ; 2,65 (dd, J = 15 et 7,5 Hz : 0,35 H) ; de 2,75 à 2,95 (mt : 1H) ; 3,01 (mt : 0,65H) ; 3,05 (dd, J = 15 et 7,5 Hz : 0,65 H) ; 3,17 (mt : 0,35 H) ; de 3,25 à 3,75 (mf : 4H) ; 3,61 (s large : 3H) ; 3,81 et 3,82 (2 s : 3H en totalité) ; de 5,00 à 5,25 (mt : 2H); 5,78 (mt : 1H).

A partir du mélange d'isomères A+B obtenus précédemment, la séparation des deux couples d'isomères s'effectue par HPLC sur une phase stationnaire Kromasil C8 à partir de 196,59 g du mélange A+B décrit précédemment, (colonne préparative; granulométrie 10 µmm ; diamètre 80 mm ; masse de la phase stationnaire 1,2 kg), sous une pression de 600 kPa, la phase mobile est composée d'un mélange de acétone-eau distillée-méthanol (60/30/10 en volumes) ayant un débit de 126 cm³ par minute et la longueur d'onde du détecteur UV est fixée à 215 nm. Les fractions contenant le premier isomère A (racémique) sont réunies et évaporée sous pression réduite (2 kPa) à une température voisine de 40°C , on obtient 32,43g d'isomère A sous forme d'une huile. Les fractions contenant le deuxième isomère noté B (racémique) sont réunies et évaporées sous pression réduite (2 kPa) à une température voisine de 40°C , on obtient 35,25 g d'isomère B sous forme d'une huile.

### Isomère A (racémique)

Spectre de R.M.N. ¹H (300 MHz, (CD₃)₂SO d6, δ en ppm) : 1,41 (s : 9H) ; 1,86 (mt : 1H) ; 2,33 (mt : 1H) ; 2,87 (dd large, J = 14,5 et 7,5 Hz : 1H) ; de 2,95 à 3,10 (mt : 2H) ; de 3,25 à 3,75 (mf étalé : 4H) ; 3,62 (s large : 3H) ; 3,81 (s : 3H) ; de 5,10 à 5,25 (mt : 2H) ; 5,80 (mt : 1H) .
IC: m/z 386 (M+H)⁺, m/z 403 (M+NH₄)⁺
Condition HPLC : colonne préparative, Kromasil C8, débit 1 cm³/min, condition d'élution
de 0 à 10 min : acétonitrile-eau distillée (60/40)
Temps de rétention : 7,39 min

### Isomère B (racémique)

Spectre de R.M.N. ¹H (300 MHz, (CD₃)₂SO d6, δ en ppm) : 1,39 (s : 9H) ; 2,18 (mt : 2H) ; 2,66 (dd, J = 15 et 7,5 Hz : 1H) ; 2,83 (dd, J = 15 et 7 Hz : 1H) ; de 2,85 à 3,10 (mt : 1H) : 3,18 (mt : 1H) ; de 3, 30 à 3,55 (mf : 1H) ; 3,66 (s très large : 3H) ; de 3,75 à 3,95 (mf : 1H) ; 3,83 (s : 3H) ; 4,00 (d très large, J = 13,5 Hz : 1H) ; 5,07 (dd, J = 18 et 1,5 Hz : 1H) ; 5,15 (dd, J = 10,5 et 1,5 Hz : 1H) ; 5,75 (mt : 1H).
IC: m/z 386 (M+H)⁺ (pic de base), m/z 403 (M+NH₄)⁺
Condition HPLC : colonne préparative, Kromasil C8, débit 1 cm³/min, condition d'élution
de 0 à 10 min : acétonitrile-eau distillée (60/40)
Temps de rétention : 7,98 min

### Exemple 2

### Synthèse des 4 stéréoisomères de l'acide (3RS,4RS)-4-[3-(R,S)-hydroxy-3-(3-chloro-6-méthoxyquinolin-4-yl)-propyl]-1-[2-(2-thiénylsulfanyl)éthyl]-pipéridine 3-carboxylique

Acide (3R,4R)-4-[-(3R)-hydroxy-3-(3-chloro-6-méthoxyquinolin-4-yl)-propyl]-1-[2-(2-thiénylsulfanyl)éthyl]-pipéridine 3-carboxylique
Acide (3R,4R)-4-[-(3S)-hydroxy-3-(3-chloro-6-méthoxyquinolin-4-yl)-propyl]-1-[2-(2-thiénylsulfanyl)éthyl]-pipéridine 3-carboxylique
Acide (3S,4S)-4-[-(3R)-hydroxy-3-(3-chloro-6-méthoxyquinolin-4-yl)-propyl]-1-[2-(2-thiénylsulfanyl)éthyl]-pipéridine 3-carboxylique
Acide (3S,4S)-4-[-(3S)-hydroxy-3-(3-chloro-6-méthoxyquinolin-4-yl)-propyl]-1-[2-(2-thiénylsulfanyl)éthyl]-pipéridine 3-carboxylique

Les quatre stéréoisomères sont nommés ci-après A, B, C, et D. Leurs stéréochimies absolues ne sont pas connues.

### Stéréoisomère A

A 480 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle (ester isomère A) solubilisé dans 10 cm³ de dioxane, on ajoute 2,7 cm³ d'une solution aqueuse d'hydroxyde de sodium 1N. Le milieu réactionnel est ensuite chauffé à 70°C pendant 5h30. On laisse ensuite revenir la température à 19°C pendant 12 heures. On évapore sous pression réduite (20 kPa ; 45°C). On reprend le résidu dans 25 cm³ d'eau distillée puis on extrait avec 25 cm³ de d'éther diéthylique. La phase aqueuse est acidifiée avec 2,6 cm³ d'une solution aqueuse d'acide chlorhydrique 1N (pH=6) puis on extrait cette phase avec 3 fois 70 cm³ d'acétate d'éthyle. La phase organique est séchée sur sulfate de magnésium, filtrée sur verre fritté puis évaporée sous pression réduite (20 kPa ; 45 °C). Après avoir séché sous vide (50 kPa) pendant 4 heures, on obtient 360 mg d'acide 4-[3-hydroxy-3-(3-chloro-6-méthoxyquinolin-4-yl)-pro.pyll-l-[2-(2-thiénylsulfanyl)éthyl]-pipéridine 3-carboxylique sous forme d'un solide jaune pâle ( isomère A).
Spectre de R.M.N. ¹H (300 MHz, (CD₃)₂SO d6, δ en ppm) : 1,34 (mt : 1H) ; de 1,45 à 1,90 (mt : 5H) ; de 2,00 à 2,15 (mt : 1H) ; de 2,15 à 2,35 (mt : 1H) ; 2,40 (d très large, J = 10,5 Hz : 1H) ; de 2,45 à 2,60 (mt : 1H) ; 2,58 (t, J = 7,5 Hz : 2H) ; de 2,60 à 2,95 (mf : 2H) ; 2,96 (mt : 2H) ; 3,89 (s : 3H) ; 5,47 (mt : 1H) ; 6,09 (mf : 1H) ; 7,07 (dd, J = 5,5 et 3,5 Hz : 1H) ; 7,21 (dd, J = 3,5 et 1 Hz : 1H) ; 7,44 (dd, J = 9 et 3 Hz : 1H) ; 7,64 (dd, J = 5,5 et 1 Hz : 1H) ; 7,96 (d, J = 9 Hz : 1H) ; 8,20 (d, J = 3 Hz : 1H) ; 8,65 (s : 1H) .
α_{D}²⁰= 28,2° +/-0,9 dans le méthanol à 0,5%

### Stéréoisomère B

A 478 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle (ester isomère B) solubilisé dans 10 cm³ de dioxane, on ajoute 2.7 cm³ d'une solution aqueuse d'hydroxyde de sodium 1N. Le milieu réactionnel est ensuite chauffé à 70°C pendant 5h30. On laisse ensuite revenir la température à 19°C pendant 12 heures. On évapore sous pression réduite (20 kPa ; 45°C) . On reprend le résidu dans 25 cm³ d'eau distillée puis on extrait avec 25 cm³ de d'éther diéthylique. La phase aqueuse est acidifiée avec 2.6 cm³ d'une solution aqueuse d'acide chlorhydrique 1N (pH=6) puis on extrait cette phase avec 3 fois 70 cm³ d'acétate d'éthyle. La phase organique est séchée sur sulfate de magnésium, filtrée sur verre fritté puis évaporée sous pression réduite (20 kPa ; 45 °C). Après avoir séché sous vide (50 kPa), on reprend le résidu avec 25 cm³ d'acétone puis on concentre à nouveau sous pression réduite (20 kPa ; 45 °C). On sèche sous pression réduite (50 kPa ; 20 °C) pendant 4 heures et on obtient 350 mg d'acide 4-[3-hydroxy-3-(3-chloro-6-méthoxyquinolin-4-yl)-propyl]-1-[2-(2-thiénylsulfanyl)éthyl]-pipéridine 3-carboxylique sous forme d'un solide jaune pâle ( isomère B).
Spectre de R.M.N. ¹H (300 MHz, (CD₃)₂SO d6, δ en ppm) : 1,32 (mt : 1H) ; de 1,45 à 1,90 (mt : 5H) ; de 2,00 à 2,15 (mt : 1H) ; de 2,15 à 2,35 (mt : 1H) ; 2,37 (d très large, J = 10,5 Hz : 1H) ; de 2,45 à 2,60 (mt : 1H) ; 2,59 (t, J = 7,5 Hz : 2H) ; de 2,65 à 3,00 (mt : 2H) ; 2,96 (mt : 2H) ; 3,90 (s : 3H) ; 5, 46 (mt : 1H) ; 6,05 (mf : 1H) ; 7,07 (dd, J = 5,5 et 3,5 Hz : 1H) ; 7,21 (dd, J = 3,5 et 1 Hz : 1H) ; 7,43 (dd, J = 9 et 3 Hz : 1H) ; 7,64 (dd, J = 5, 5 et 1 Hz : 1H) ; 7,95 (d, J = 9 Hz : 1H) ; 8,20 (d, J = 3 Hz : 1H) ; 8,65 (s : 1H) ; de 12,80 à 13,40 (mf étalé : 1H).
α_{D}²⁰= -25,2° +/- 1,5 dans le méthanol à 0,5%

### Stéréoisomère C

A 300 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle (ester isomère C) solubilisé dans 10 cm³ de dioxane, on ajoute 1,7 cm³ d'une solution aqueuse d'hydroxyde de sodium 1N. Le milieu réactionnel est ensuite chauffé à 70°C pendant 5h30. On laisse revenir la température à 19°C pendant 12 heures puis on chauffe à nouveau à 70 °C pendant 2 heures. On évapore ensuite sous pression réduite ( 20 kPa ; 45°C). On reprend le résidu dans 25 cm³ d'eau distillée puis on extrait avec 25 cm³ de d'éther diéthylique. La phase aqueuse est acidifiée avec 1,6 cm³ d'une solution aqueuse d'acide chlorhydrique 1N (pH=6) puis on extrait cette phase avec 3 fois 70 cm³ d'acétate d'éthyle. La phase organique est séchée sur sulfate de magnésium, filtrée sur verre fritté puis évaporée sous pression réduite (20 kPa ; 45 °C). Après avoir séché sous vide (50 kPa), on reprend le résidu avec 20 cm³ d'acétone puis on concentre à nouveau sous pression réduite (20 kPa ; 45 °C). On sèche sous pression réduite (50 kPa ; 20 °C) pendant 12 heures et on obtient 250 mg d'acide 4-[3-hydroxy-3-(3-chloro-6-méthoxyquinolin-4-yl)-propyl]-1-[2-(2-thiénylsulfanyl)éthyl]-pipéridine 3-carboxylique sous forme d'un solide jaune pâle (isomèreC).
Spectre de R.M.N. ¹H (300 MHz, (CD₃)₂SO d6, δ en ppm) : 1,30 (mt : 1H) ; de 1,45 à 1,85 (mt : 5H) ; 2,19 (mt : 2H) ; 2,37 (d très large, J = 11 Hz : 1H) ; de 2,45 à 2,80 (mt : 2H) ; 2, 58 (t, J = 7,5 Hz : 2H) ; de 2,80 à 3, 10 (mf : 1H) ; 2,96 (mt : 2H) ; 3,91 (s : 3H) ; 5,45 (mt : 1H) ; 6,11 (mf étalé : 1H) ; 7,07 (dd, J = 5,5 et 3,5 Hz : 1H) ; 7,22 (dd, J = 3, 5 et 1 Hz : 1H) ; 7,43 (dd, J = 9 et 3 Hz : 1H) ; 7,65 (dd, J = 5,5 et 1 Hz : 1H) ; 7,95 (d, J = 9 Hz : 1H) ; 8,24 (d, J = 3 Hz : 1H) ; 8,65 (s : 1H) .
α_{D}²⁰= 88,1° +/- 1,5 dans le méthanol à 0,5%

### Stéréoisomère D

A 325 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle (ester isomère D) solubilisé dans 10 cm³ de dioxane, on ajoute 1.8 cm³ d'une solution aqueuse d'hydroxyde de sodium 1N. Le milieu réactionnel est ensuite chauffé à 70°C pendant 5h30. On laisse revenir la température à 19°C pendant 12 heures puis on chauffe à nouveau à 70 °C pendant 2 heures. On évapore ensuite sous pression réduite ( 20 kPa ; 45°C) . On reprend le résidu dans 25 cm³ d'eau distillée puis on extrait avec 25 cm³ de d'éther diéthylique. La phase aqueuse est acidifiée avec 1.6 cm³ d'une solution aqueuse d'acide chlorhydrique 1N (pH=6) puis on extrait cette phase avec 3 fois 70 cm³ d'acétate d'éthyle. La phase organique est séchée sur sulfate de magnésium, filtrée sur verre fritté puis évaporée sous pression réduite (20 kPa ; 45 °C). Après avoir séché sous vide (50 kPa), on reprend le résidu avec 20 cm³ d'acétone puis on concentre à nouveau sous pression réduite (20 kPa ; 45 °C). On sèche sous pression réduite (50 kPa ; 20 °C) pendant 12 heures et on obtient 260 mg d'acide 4-[3-hydroxy-3-(3-chloro-6-méthoxyquinolin-4-yl)-propyl]-1-[2-(2-thiénylsulfanyl)éthyl]-pipéridine 3-carboxylique sous forme d'un solide jaune pâle (isomère D).
Spectre de R.M.N. ¹H (300 MHz, (CD₃)₂SO d6, δ en ppm) : 1,30 (mt : 1H) ; de 1,40 à 1,85 (mt : 5H) ; 2,19 (mt : 2H) ; 2,37 (d très large, J = 10,5 Hz : 1H) ; 2,58 (t, J = 7,5 Hz : 2H) ; de 2,60 à 2,75 (mt : 1H) ; de 2,80 à 3,05 (mt : 1H) ; 2,96 (mt : 2H) ; 3,90 (s : 3H) ; 5,45 (mt : 1H) ; 6,09 (mt : 1H) ; 7,07 (dd, J = 5,5 et 3,5 Hz : 1H) ; 7,22 (dd, J = 3,5 et 1 Hz : 1H) ; 7,43 (dd, J = 9 et 3 Hz : 1H) ; 7,64 (dd, J = 5,5 et 1 Hz : 1H) ; 7,95 (d, J = 9 Hz : 1H) ; 8,23 (d, J = 3 Hz : 1H) ; 8,65 (s : 1H) .
α_{D}²⁰= -88,1° +/- 1,5 dans le méthanol à 0,5%
Synthèse des 4 stéréoisomères du (3RS,4RS)-4-[3-(R,S)-hydroxy-3-(3-chloro-6-méthoxyquinolin-4-yl)-propyl]-1-[2-(2-thiénylsulfanyl)éthyl]-pipéridine 3-carboxylate de méthyle
(3R,4R)-4-[(3R)-hydroxy-3-(3-chloro-6-méthoxyquinolin-4-yl)-propyl]-1-[2-(2-thiénylsulfanyl)éthyl]-pipéridine 3-carboxylate de méthyle
(3R,4R)-4-[(3S)-hydroxy-3-(3-chloro-6-méthoxyquinolin-4-yl)-propyl]-1-[2-(2-thiénylsulfanyl)éthyl]-pipéridine 3-carboxylate de méthyle
(3S,4S)-4-[(3R)-hydroxy-3-(3-chloro-6-méthoxyquinolin-4-yl)-propyl]-1-[2-(2-thiénylsulfanyl)éthyl]-pipéridine 3-carboxylate de méthyle
(3S,4S)-4-[(3S)-hydroxy-3-(3-chloro-6-méthoxyquinolin-4-yl)-propyl]-1-[2-(2-thiénylsulfanyl)éthyl]-pipéridine 3-carboxylate de méthyle

Les quatre stéréoisomères sont nommés ci-après A, B, C, et D. Leurs stéréochimies absolues ne sont pas connues.

A 2,5 g de chlorhydrate de (3RS, 4RS)-4-[-3-(R,S)-hydroxy-3-(3-chloro-6-méthoxyquinolin-4-yl)prapyl]-1-H-pipéridine-3-carboxylate de méthyle solubilisé dans 110 cm³ d'acétonitrile, on ajoute 1,5 cm³ de triéthylamine, 2,15 g de carbonate de potassium et 0,85 g d'iodure de potassium. Toujours à 20°C, on ajoute 1,15 g de 2-(bromoéthylsulfanyl)-thiophène. Le milieu réactionnel est ensuite porté à 60 °C pendant 16 heures. On laisse ensuite revenir le milieu à 20 °C puis on l'évapore sous pression réduite (45 °C ; 5 kPa). Le résidu est repris avec 200 cm³ d'acétate d'éthyle et 100 cm³ d'eau distillée. La phase organique est relavée avec 2 fois 100 cm³ d'une solution aqueuse saturée en chlorure de sodium, séchée sur sulfate de magnésium pendant 1 heure, filtrée sur verre frittée puis évaporée sous pression réduite (45 °C ; 5 kPa) Le résidu est purifié par chromatographie, sous une pression d'azote de 50 kPa, sur une colonne de gel de silice (granulométrie 0,065-0,2 µ ; diamètre 2,5 cm ; hauteur 35 cm), en éluant par un mélange de cyclohexane-acétate d'éthyle (60/40 en volumes) et en recueillant des fractions de 50 cm³. Les fractions 6 à 9 sont réunies, puis concentrées sous pression réduites (45 °C ; 5 kPa). On obtient 1,95 g de (3RS,4RS)-4-[3-(R,S)-hydroxy-3-(3-chloro-6-méthoxyquinolin-4-yl)-propyl]-1-[2-(2-thiénylsulfanyl)éthyl]-pipéridine 3-carboxylate de méthyle (mélange des isomères A, B, C, D) sous forme d'une huile incolore.

Le 2-(bromoéthylsulfanyl)-thiophène peut être préparé selon le brevet W0200125227.
Spectre de R.M.N. ¹H (300 MHz, (CD₃)₂SO d6, δ en ppm) . On observe un mélange de 2 diastéréoisomères dans les proportions 60/40.
* de 1,10 à 1,85 (mt : 7H) ; de 1,85 à 2,85 (mt : 7H) ; 2,89 (t large, J = 7,5 Hz : 2H) ; 3,42 et 3,56 (2 s : 3H en totalité) ; 3,89 et 3,90 (2 s : 3H en totalité) ; 5,45 (mt : 1H) ; 6,01 (s large : 1H) ; 7,05 (dd, J = 5,5 et 3,5 Hz : 1H) ; 7,18 (dd, J = 3,5 et 1,5 Hz : 1H) ; 7,44 (dd, J = 9 et 3 Hz : 1H) ; 7,61 (dd, J = 5,5 et 1,5 Hz : 1H) ; 7,95 (d, J = 9 Hz : 1H) ; 8,19 (mt : 1H) ; 8,65 et 8,66 (2 s : 1H en totalité).

IE: m/z 534 (M⁺), m/z 504 (pic de base)

A partir du mélange de stéréoisomères A,B,C,D obtenu précédemment, la séparation de chaque stéréoisomère s'effectue par HPLC.

La séparation des 2 couples de stéréoisomères (A+B) et (C+D), est réalisée sur une phase stationnaire Symmetry C18 à partir de 1,95 g du mélange A,B,C,D décrit précédemment, ( granulométrie 7 µmm ; diamètre 60 mm ; masse de la phase stationnaire 700 g), sous une pression de 500 kPa, la phase mobile est composée d'un mélange de méthanol-solution tampon aqueuse (pH=4.9)-acétonitrile (10/55/35 en volumes) ayant un débit de 120 cm³ par minute et la longueur d'onde du détecteur UV est fixée à 280 nm. Les fractions contenant la première paire d'énantiomères notée (A+B) sont réunies et évaporées sous pression réduite (2 kPa) à une température voisine de 40°C. Le résidu obtenu est repris dans de.l'eau puis extrait avec 2 fois du dichlorométhane. La phase organique est séchée sur sulfate de magnésium, filtrée puis évaporée sous pression réduite (2 kPa ; 45 °C). On obtient 640 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle (mélange A+B).

Les fractions contenant la deuxième paire d'énantiomères notée (C+D) sont réunies et évaporées sous pression réduite (2 kPa) à une température voisine de 40°C. Le résidu obtenu est repris dans de l'eau puis extrait avec 2 fois du dichlorométhane. La phase organique est séchée sur sulfate de magnésium, filtrée puis évaporée sous pression réduite (2 kPa ; 45 °C).On obtient 620 mg de 4-[3-hydroxy-3-(3-chloro-6-méthoxyquinolin-4-yl)-propyl]-1-[2-(2-thiénylsulfanyl)éthyl]-pipéridine 3-carboxylate de méthyle ( mélange C+D).

Ensuite les produits du couple d'énantiomères (A,B) sont séparés sur une colonne chiracel OJ (granulométrie 20 µmm ; diamètre 35 mm ; masse de la phase stationnaire 700 g) sous une pression de 1510 kPa, la phase mobile est composée d'un mélange de heptane-éthanol-triéthylamine (90/10/0.1 en volumes) ayant un débit de 120 cm³ par minute et la longueur d'onde du détecteur UV est fixée à 254 nm. Les fractions contenant chaque produit sont isolées puis concentrées sous une pression réduite (2 kPa) à une température voisine de 40°C ; on obtient 0,48 g du stéréoisomère A et 0,478 g du stéréoisomère B.

De même, les produits du couple d'énantiomères (C,D) sont séparés sur une colonne chiracel OD (granulométrie 20 µmm ; diamètre 80 mm ; masse de la phase stationnaire 1250 g) sous une pression de 1510 kPa, la phase mobile est composée d'un mélange de heptane-isopropanol-méthanol-triéthylamine (93/4/3/0.1 en volumes) ayant un débit de 150 cm³ par minute et la longueur d'onde du détecteur UV est fixée à 265 nm. Les fractions contenant chaque produit sont isolées puis concentrées sous une pression réduite (2 kPa) à une température voisine de 40°C ; on obtient 0,30 g du stéréoisomère C sous forme d'un solide blanchâtre , et 0,325 g du stéréoisomère D sous forme d'un solide blanchâtre.

### Stéréoisomère A

Spectre de R.M.N. ¹H (400 MHz, (CD₃)₂SO d6, δ en ppm) : 1,30 (mt : 1H) ; 1, 53 (mt : 1H) ; de 1,65 à 1,85 (mt : 4H) ; 2,10 (mt : 1H) ; 2,22 (mt : 1H) ; 2,39 (dd, J = 12 et 4 Hz : 1H) ; de 2,45 à 2,60 (mt : 3H) ; 2,64 (mt : 1H) ; 2,73 (dd, J = 12 et 6,5 Hz : 1H) ; 2,91 (t, J = 7 Hz : 2H) ; 3,45 (s : 3H) ; 3,92 (s : 3H) ; 5,49 (mt : 1H) ; 5,79 (mf : 1H) ; 7,04 (dd, J = 5,5 et 3,5 Hz : 1H) ; 7,16 (dd, J = 3,5 et 1 Hz : 1H) ; 7,43 (dd, J = 9 et 3 Hz : 1H) ; 7,56 (dd, J = 5, 5 et 1 Hz : 1H) ; 7,96 (d, J = 9 Hz : 1H) ; 8,21 (d, J = 3 Hz : 1H) ; 8,63 (s : 1H) .
α_{D}²⁰= -28,8° +/- 0,7 dans le dichlorométhane à 0,5%
Condition HPLC : colonne Chiralcel OJ, débit 1 cm³/min, condition d'élution
de 0 à 35 mim : éthanol-heptane-triéthylamine (10/90/0.1 en volumes)
Temps de rétention : 18,54 min

### Stéréoisomère B

Spectre de R.M.N. ¹H (300 MHz, (CD₃)₂SO d6, δ en ppm) : de 1,05 à 1,35 (mt : 1H) ; 1, 51 (mt : 1H) ; 1,72 (mt : 4H) ; de 2,00 à 2,25 (mt : 2H) ; 2,32 (d large, J = 11 Hz : 1H) ; de 2,40 à 2,55 (mt : 3H) ; 2,64 (mt : 1H) ; 2,73 (mt : 1H) ; 2,89 (t, J = 7,5 Hz : 2H); 3,41 (s : 3H) ; 3,88 (s : 3H) ; 5,45 (mt : 1H) ; 6,03 (d, J = 3,5 Hz : 1H) ; 7,05 (dd, J = 5, 5 et 3, 5 Hz : 1H) ; 7,18 (dd, J = 3, 5 et 1 Hz : 1H) ; 7,44 (dd, J = 9 et 3 Hz : 1H) ; 7,62 (dd, J = 5, 5 et 1 Hz : 1H) ; 7,96 (d, J = 9 Hz : 1H) ; 8,19 (d, J = 3 Hz : 1H) ; 8,65 (s : 1H).
α_{D}²⁰= -31,7° +/- 0,8 dans le dichlorométhane à 0.5%
Condition HPLC : colonne Chiralcel OJ, débit 1 cm³/min, condition d'élution
de 0 à 35 mim : éthanol-heptane-triéthylamine (10/90/0.1 en volumes)
Temps de rétention : 24,31 min

### Stéréoisomère C

Spectre de R.M.N. ¹H (300 MHz, (CD₃)₂SO d6, δ en ppm) : 1,43 (mt : 2H) ; de 1,55 à 1,85 (mt : 4H) ; 1,96 (mt : 1H) ; 2,13 (mt : 1H) ; 2,32 (d très large, J = 11 Hz : 1H) ; de 2,35 à 2,60 (mt : 3H) ; 2,67 (mt : 1H) ; 2,76 (mt : 1H) ; 2,88 (t, J = 7,5 Hz : 2H) ; 3,56 (s : 3H) ; 3,89 (s : 3H) ; 5,45 (mt : 1H) ; 6,02 (d, J = 3,5 Hz : 1H) ; 7,05 (dd, J = 5, 5 et 3,5 Hz : 1H) ; 7,17 (dd, J = 3, 5 et 1 Hz : 1H) ; 7,44 (dd, J = 9 et 3 Hz : 1H) ; 7,61 (dd, J = 5,5 et 1 Hz : 1H) ; 7,95 (d, J = 9 Hz : 1H) ; 8,19 (d, J = 3 Hz : 1H) ; 8,66 (s : 1H).
α_{D}²⁰= 27,8° +/- 0,8 dans le DMSO à 0,5%
Condition HPLC : colonne Chiralcel OD, débit 1 cm³/min, condition d'élution
de 0 à 35 mim : heptane-isopropanol-éthanol-triéthylamine (93/4/3/0.1 en volumes)
Temps de rétention : 16,19 min

### Stéréoisomère D

Spectre de R.M.N. ¹H (300 MHz, (CD₃)₂SO d6, δ en ppm) : 1, 44 (mt : 2H) ; de 1, 55 à 1,85 (mt : 4H) ; 1,97 (mt : 1H) ; 2,14 (mt : 1H) ; 2,32 (d très large, J = 11 Hz : 1H) ; de 2,35 à 2,60 (mt : 3H) ; 2,67 (mt : 1H) ; 2,76 (mt : 1H) ; 2,88 (t, J = 7,5 Hz : 2H) ; 3,56 (s : 3H) ; 3,89 (s : 3H) ; 5,44 (mt : 1H) ; 6,03 (d, J = 4 Hz : 1H) ; 7,06 (dd, J = 5,5 et 3,5 Hz : 1H) ; 7,18 (dd, J = 3,5 et 1 Hz : 1H) ; 7,44 (dd, J = 9 et 3 Hz : 1H) ; 7,62 (dd, J = 5,5 et 1 Hz : 1H) ; 7,95 (d, J = 9 Hz : 1H) ; 8,20 (d, J = 3 Hz : 1H) ; 8,66 (s : 1H) .
α_{D}²⁰= -30,0°+/- 0,8 dans le DMSO à 0,5%
Condition HPLC : colonne Chiralcel OD, débit 1 cm³/min, condition d'élution
de 0 à 35 mim : heptane-isopropanol-éthanol-triéthylamine (93/4/3/0.1 en volumes)
Temps de rétention : 19,41 min

## Revendications

1. Un dérivé de quinolyl propyl pipéridine, **caractérisé en ce qu'**il répond à la formule générale 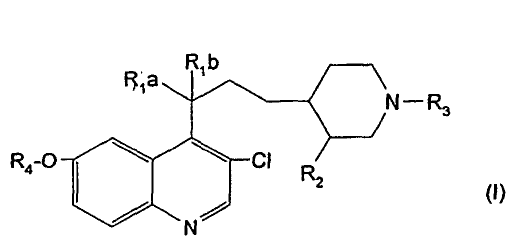
dans laquelle :
R₁ₐ est un atome d'hydrogène ou d'halogène ou un radical hydroxy, amino, alcoylamino, dialcoylamino, hydroxyamino, alcoyloxyamino ou alcoyl alcoyloxy amino et R_{1b} est un atome d'hydrogène, ou R₁ₐ et R_{1b} forment un groupement oxo,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, par un radical cycloalcoylthio dont la partie cyclique contient 3 à 7 chaînons pouvant lui-même porter 1 ou plusieurs substituants choisis parmi halogène et trifluorométhyle, ou par un radical hétéroarylthio de 5 à 6 chaînons comprenant 1 à 4 hétéroatomes choisis parmi l'azote, l'oxygène et le soufre, pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino ou R₃ représente un radical propargyle substitué par un radical phényle pouvant lui même porter 1 à 4 substituants choisis dans le groupe constitué par halogène, hydroxy, alcoyle, alcoyloxy, trifluorométhyle, trifluorométhoxy, carboxy, alcoyloxycarbonyle, cyano et amino, ou substitué par un radical cycloalcoyle contenant 3 à 7 chaînons, pouvant lui-même porter 1 ou plusieurs substituants choisis parmi halogène et trifluorométhyle, ou substitué par un radical hétéroaryle de 5 à 6 chaînons comprenant 1 à 4 hétéroatomes choisis parmi l'azote, l'oxygène et le soufre et pouvant lui-même porter un ou plusieurs substituants choisis dans le groupe constitué par halogène, hydroxy, alcoyle, alcoyloxy, trifluorométhyle, trifluorométhoxy, carboxy, alcoyloxycarbonyle, cyano et amino, et
R₄ représente un radical alcoyle contenant 1 à 6 atomes de carbone, alcényl-CH₂- ou alcynyl-CH₂- dont les parties alcényle ou alcynyle contiennent 2 à 6 atomes de carbone, cycloalcoyle ou cycloalcoyl alcoyle dont la partie cycloalcoyle contient 3 à 8 atomes de carbone,
sous ses différentes forme isomères, énantiomères et diastéréoisomères, séparées ou en mélanges, ainsi que ses sels.

2. Un dérivé de formule (I) telle que définie à la revendication 1, dans laquelle R₁ₐ est un radical hydroxy et R₂ₐ est un atome d'hydrogène.

3. Un dérivé de formule (I) telle que définie à la revendication 1, dans laquelle R₁ₐ et R₂ₐ forment un groupement oxo.

4. Un dérivé de formule (I) tellen que définie à l'une quelconque des revendications 1 à 3, dans laquelle R₄ représente un radical alcoyle contenant de 1 à 6 atomes de carbone.

5. Un dérivé de formule (I) telle que définie à l'une quelconque des revendications 1 à 4, dans laquelle R₂ représente un radical carboxy.

6. Un dérivé de formule (I) telle que définie à l'une quelconque des revendications 1 à 5, dans laquelle R₃ représente un radical alcoyle renfermant de 1 à 6 atomes de carbone, substitué par un radical phénylthio, cycloalcoylthio ou hétéroarylthio éventuellement substitué, tel que défini-à la revendication 1.

7. Un dérivé de formule (I) telle que définie à l'une quelconque des revendications 1 à 6, dans laquelle R₃ représente un radical éthyl substitué par un radical thiénylthio, phénylthio substitué par halogène, cyclohexylthio ou cyclopentylthio.

8. L'un quelconque des dérivés de formule générale (I) telle que définie à à la revendication 1, dont les noms suivent :
L'acide -4-[3-hydroxy-3-(3-chloro-6-méthoxyquinolin-4-yl)-propyl]-1-[2-(2,5-difluoro-phénylsulfanyl)-éthyl]-pipéridine-3-carboxylique ;
L'acide -4-[3-hydroxy-3-(3-chloro-6-méthoxyquinolin-4-yl)-propyl]-1-[2-(2-thiénylsulfanyl)-éthyl]-pipéridine-3-carboxylique ;
sous ses différentes formes isomères, séparées ou en mélanges, ainsi que ses sels.

9. Un procédé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale 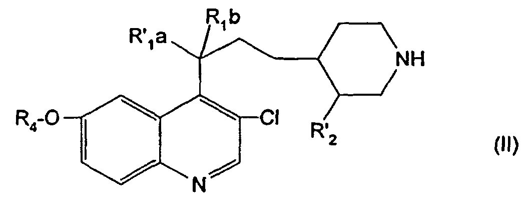
dans laquelle R₄ est défini comme dans la revendication 1, soit R'₁ₐ représente un atome d'hydrogène ou un radical hydroxy et R_{1b} représente un atome d'hydrogène soit R'₁ₐ et R_{1b} forment un groupement oxo et R'₂ représente un radical carboxy ou carboxyméthyle protégé, pour obtenir un dérivé de quinolyl propyl pipéridine de formule générale : 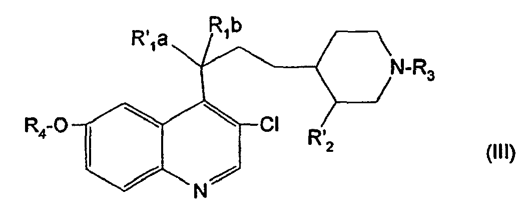
dans laquelle R'₁ₐ, R_{1b}, R'₂, R₃ et R₄ sont définis comme ci-dessus,
puis, le cas échéant, traite le dérivé pour lequel R'₁ₐ est un radical hydroxy et R_{1b} est un atome d'hydrogène par un réactif d'halogénation,
ou bien, le cas échéant, transforme par oxydation le radical hydroxy représenté par R'₁ₐ en un radical oxo, puis, le cas échéant, transforme celui-ci en un radical hydroxyimino ou alcoyloxyimino, selon les méthodes connues, pour obtenir un dérivé de quinolyl propyl pipéridine de formule générale : 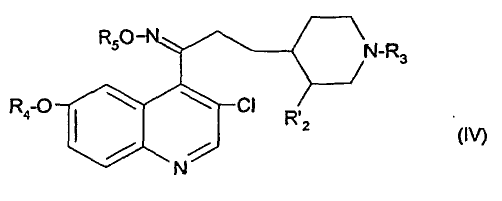
pour lequel R'₂, R₃ et R₄ sont définis comme précédemment, et R₅ est un atome d'hydrogène ou un radical alcoyle, et réduit le dérivé de formule générale (IV) pour lequel R₅ est un atome d'hydrogène en amine, et le cas échéant transforme en une amine monoalcoylée ou dialcoylée, ou le cas échéant réduit le dérivé de formule générale (IV) pour lequel R₅ est un atome d'hydrogène en hydroxylamine, ou le dérivé de formule générale (IV) pour lequel R₅ est un radical alcoyle en alcoyloxyamine, puis, le cas échéant, pour obtenir le dérivé pour lequel R₁ₐ est alcoyl alcoyloxy amino, transforme le dérivé obtenu pour lequel R₁ₐ est alcoyloxyamino par alcoylation,
puis transforme R'₂ en un radical carboxy ou carboxyméthyl, et/ou, le cas échéant, réduit le radical carboxy ainsi obtenu ou le radical carboxy protégé que peut représenter R'₂ en un radical hydroxyméthyle et le cas échéant transforme celui-ci en un radical carboxyméthyle selon les méthodes habituelles,
puis, le cas échéant, sépare les isomères, le cas échéant élimine le radical protecteur d'acide, et/ou, le cas échéant, transforme le produit obtenu en un sel.

10. Procédé selon la revendication 9, **caractérisé en ce que** l'on prépare le dérivé de quinolyl propyl pipéridine de formule générale (II), pour lequel R'₁ₐ est un atome d'hydrogène par oxydation en milieu basique d'un dérivé correspondant pour lequel R'1a et R_{1b} sont des atomes d'hydrogène, la fonction amine de la pipéridine est protégée intermédiairement et R'₂ est tel que défini précédemment ou représente un radical carboxy ou carboxyméthyle, et, le cas échéant, reprotection du radical carboxy ou carboxyméthyle.

11. Procédé selon la revendication 9, **caractérisé en ce que** l'on prépare le dérivé de quinolyl propyl pipéridine de formule générale (II) dans laquelle R'₁ₐ et R_{1b} forment un groupement oxo par oxydation selon les méthodes connues d'un dérivé de formule générale (II) dans laquelle R'₁ₐ représente un radical hydroxy, obtenu comme décrit à la revendication 10.

12. Procédé selon la revendication 9, **caractérisé en ce que** l'on prépare le dérivé de quinolyl propyl pipéridine de formule générale (II) pour lequel R'₂ représente un radical carboxyméthyle protégé, et R'₁ₐ et R_{1b} sont des atomes d'hydrogène, par un procédé selon lequel l'on condense par réaction de Witting un ylure de phosphore approprié sur un dérivé de pipéridine de formule générale : 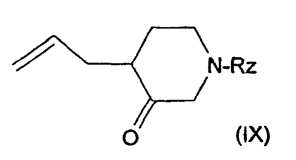
dans laquelle Rz represente un radical protecteur d'amino, pour obtenir un dérivé de formule 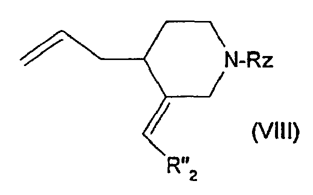
dans laquelle Rz est défini comme ci-dessus et R"₂ représente un radical carboxy protégé, que l'on condense sur un dérivé de quinoléine de formule générale : 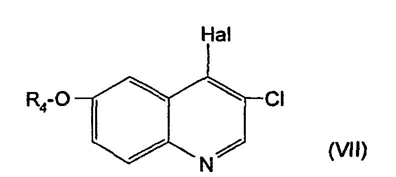
dans laquelle R4 est défini comme dans la revendication 1 et Hal représente un atome d'iode ou de brome, pour obtenir un dérivé de quinolyl propyl pipéridine de formule générale : 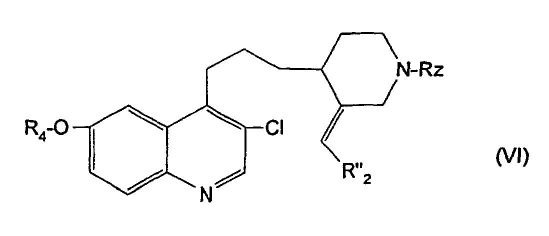
dans laquelle R"₂ et Rz sont définis comme précédemment que l'on soumet à une hydrogénation sélective et, le cas échéant, à une réaction de déprotection de l'amino.

13. Procédé selon la revendication 12, **caractérisé en ce que** l'on soumet le composé de formule (II) à une réduction du radical carboxyméthyle protégé en un radical hydroxyéthyle, transformation de celui-ci en un dérivé p-toluènesulfonyloxyéthyle, puis transformation de ce dérivé en dérivé vinylique par réaction d'élimination suivie de l'oxydation du dérivé obtenu et de l'introduction du groupement protecteur sur le radical carboxy ainsi obtenu.

14. Procédé selon la revendication 9, **caractérisé en ce que** l'on prépare le dérivé de quinolyl propyl pipéridine de formule générale (II), pour lequel R'₁ₐ et R_{1b} sont des atomes d'hydrogène, par allylation du cétoester de formule générale (XIV) 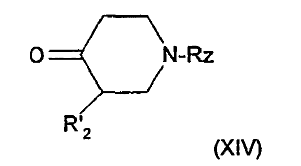
pour laquelle R'₂ est défini comme dans la revendication 8 et Rz est défini comme dans la revendication 12, pour obtenir un dérivé de formule générale (XIII) : 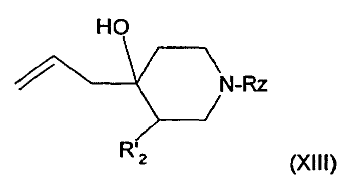
dans laquelle R'₂ et Rz sont définis comme précédemment, que l'on fait agir avec un halogènure d'alkyloxalyle pour obtenir un dérivé de formule générale (XII) : 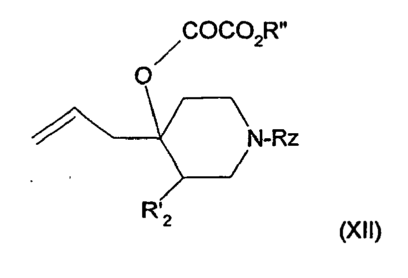
dans laquelle R" représente un radical alkyle et R'₂ et Rz sont définis comme précédemment, que l'on soumet à une réaction de déoxygénation radicalaire, pour obtenir un dérivé de formule générale (X) : 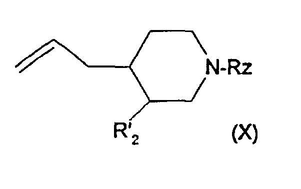
dans laquelle R'₂ et Rz sont définis comme précédemment, que l'on condense avec un dérivé de quinoléine de formule générale (VII) telle que définie à la revendication 10, pour obtenir un dérivé de formule générale (XI) : 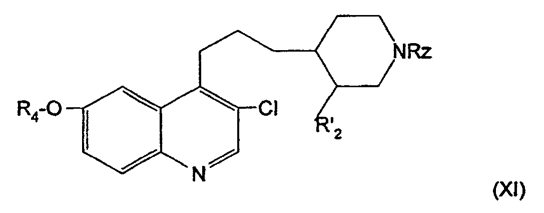
puis élimine le radical Rz protecteur d'amino.

15. A titre de médicaments, les dérivés de quinolyl propylpipéridine tels que définis à la revendication 1 ainsi que leurs sels pharmaceutiquement acceptables.

16. A titre de médicaments, les dérivés de quinolyl propylpipéridine tels que définis à l'une quelconque des revendications 2 à 8, ainsi que leurs sels pharmaceutiquement acceptables.

17. Compositions pharmaceutiques contenant, à titre de principe actif, au moins un médicament selon l'une des revendications 15 et 16.

18. Un dérivé de quinolyl pipéridine, **caractérisé en ce qu'**il répond à la formule générale : 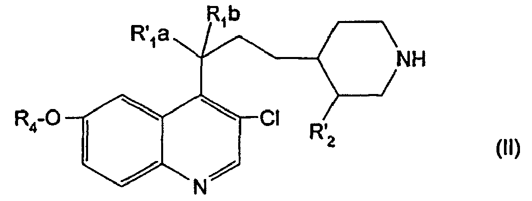
dans laquelle R₄ est défini comme dans la revendication 1,-soit R'₁ₐ représente un atome d'hydrogène ou un radical hydroxy et R_{1b} représente un atome d'hydrogène soit R'₁ₐ et R1b forment un groupement oxo et R'₂ est défini comme dans la revendication 9.

19. Un dérivé de quinolyl propyl pipéridine, **caractérisé en ce qu'**il répond à la formule générale : 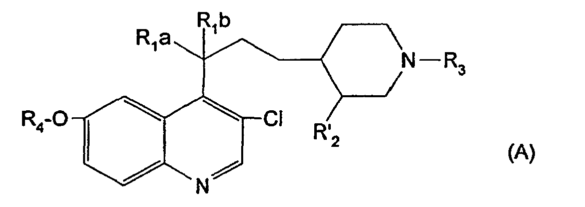
dans laquelle R₁ₐ, R_{1b}, R₃ et R₄ sont définis comme dans la revendication 1 et R'₂ est défini comme dans la revendication 9.

20. Un dérivé de quinolyl propyl pipéridine, **caractérisé en ce qu'**il répond à la formule générale : 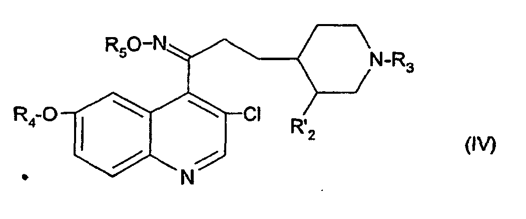
dans laquelle R₃ et R₄ sont définis comme dans la revendication 1 et R'₂ et R₅ sont définis comme dans la revendication 9.

21. Un dérivé de quinolyl propyl pipéridine, **caractérisé en ce qu'**il répond à la formule générale : 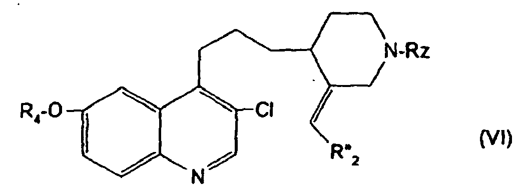
dans laquelle R₄ est défini comme dans la revendication 1 et R"₂ et Rz sont définis comme dans la revendication 12.

22. Un dérivé de quinolyl propyl pipéridine, **caractérisé en ce qu'**il répond à la formule générale : 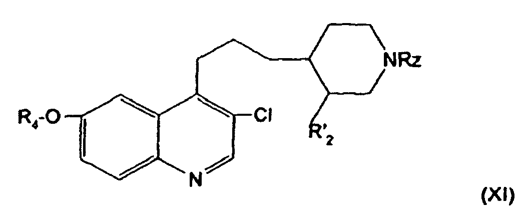
dans laquelle R4 est défini comme dans la revendication 1, R'₂ est défini comme dans la revendication 9 et Rz est défini comme dans la revendication 12.

## Claims

1. A quinolylpropylpiperidine derivative, which corresponds to the general formula 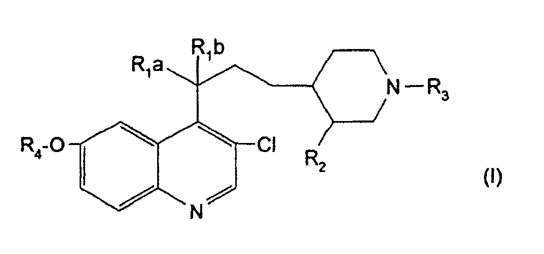
in which:
R₁ₐ is a hydrogen or halogen atom or a hydroxyl, amino, alkylamino, dialkylamino, hydroxyamino, alkyloxyamino or alkylalkyloxyamino radical, and R_{1b} is a hydrogen atom, or R₂ₐ and R_{1b} form an oxo group,
R₂ represents a carboxyl, carboxymethyl or hydroxymethyl radical,
R₃ represents an alkyl (1 to 6 carbon atoms) radical substituted with a phenylthio radical which may itself carry 1 to 4 substituents chosen from the group consisting of halogen, hydroxyl, alkyl, alkyloxy, trifluoromethyl, trifluoro-methoxy, carboxyl, alkyloxycarbonyl, cyano and amino, with a cycloalkylthio radical in which the cyclic portion contains 3 to 7 members, which may itself carry 1 or more substituents chosen from halogen and trifluoromethyl, or with a 5- to 6-membered heteroarylthio radical comprising 1 to 4 heteroatoms chosen from nitrogen, oxygen and sulfur, which may itself carry one or more substituents chosen from the group consisting of halogen, hydroxyl, alkyl, alkyloxy, trifluoromethyl, trifluoromethoxy, and carboxyl, alkyloxycarbonyl, cyano and amino or R₃ represents a propargyl radical substituted with a phenyl radical which may itself carry 1 to 4 substituents chosen from the group consisting of halogen, hydroxyl, alkyl, alkyloxy, trifluoromethyl, trifluoromethoxy, carboxyl, alkyloxycarbonyl, cyano and amino, or substituted with a 3- to 7-membered cycloalkyl radical which may itself carry one or more substituents chosen from halogen and trifluoromethyl, or substituted with a 5- to 6-membered heteroaryl radical comprising 1 to 4 heteroatoms chosen from nitrogen, oxygen and sulfur and which may itself carry one or more substituents chosen from the group consisting of halogen, hydroxyl, alkyl, alkyloxy, trifluoromethyl, trifluoromethoxy, carboxyl, alkyloxycarbonyl, cyano and amino, and
R₄ represents an alkyl radical containing 1 to 6 carbon atoms, an alkenyl-CH₂- or alkynyl-CH₂₋radical in which the alkenyl or alkynyl portions contain 2 to 6 carbon atoms, a cycloalkyl or cycloalkylalkyl radical in which the cycloalkyl portion contains 3 to 8 carbon atoms, in its various isomeric, enantiomeric and diastereoisomeric forms, separate or as mixtures, and also its salts.

2. A derivative of formula (I) as defined in claim 1, in which R₁ₐ is a hydroxyl radical and R₂ₐ is a hydrogen atom.

3. A derivative of formula (I) as defined in claim 1, in which R₁ₐ and R₂ₐ form an oxo group.

4. A derivative of formula (I) as defined in any one of claims 1 to 3, in which R₄ represents an alkyl radical containing from 1 to 6 carbon atoms.

5. A derivative of formula (I) as defined in any one of claims 1 to 4, in which R₂ represents a carboxyl radical.

6. A derivative of formula (I) as defined in any one of claims 1 to 5, in which R₃ represents an alkyl radical containing from 1 to 6 carbon atoms, substituted with an optionally substituted phenylthio, cycloalkylthio or heteroarylthio radical, as defined in claim 1.

7. A derivative of formula (I) as defined in any one of claims 1 to 6, in which R₃ represents an ethyl radical substituted with a thienylthio radical or a phenylthio radical substituted with halogen, cyclohexylthio or cyclopentylthio.

8. Any one of the derivatives of general formula (I) as defined in claim 1, with the following names:
4-[3-hydroxy-3-(3-chloro-6-methoxyquinolin-4-yl)-propyl]-1-[2-(2,5-difluorophenylsulfanyl)ethyl]piperidine-3-carboxylic acid;
4-[3-hydroxy-3-(3-chloro-6-methoxyquinolin-4-yl)-propyl]-1-[2-(2-thienylsulfanyl)ethyl]piperidine-3-carboxylic acid;
in its various isomeric forms, separate or as mixtures, and also its salts.

9. A process for preparing a quinolylpropylpiperidine derivative as claimed in claim 1, in which the R₃ chain defined in claim 1 is condensed with the quinolylpropylpiperidine derivative of general formula: 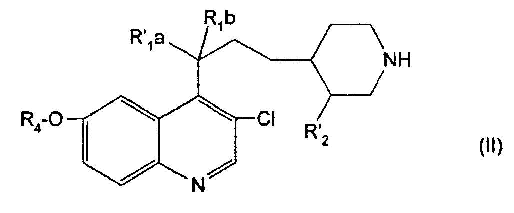
in which R₄ is as defined in claim 1, either R'₁ₐ represents a hydrogen atom or a hydroxyl radical and R_{1b} represents a hydrogen atom or R'₁ₐ and R_{1b} form an oxo group and R'₂ represents a protected carboxyl or carboxymethyl radical, to obtain a quinolylpropylpiperidine derivative of general formula: 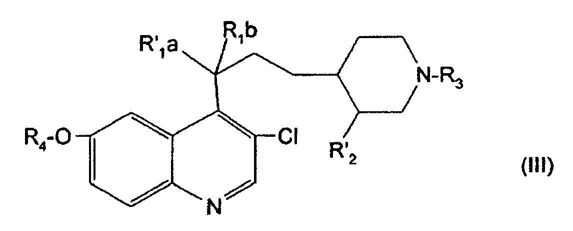
in which R'₁ₐ, R_{1b}, R'₂, R₃ and R₄ are as defined above,
and then, where appropriate, the derivative for which R'₁ₐ is a hydroxyl radical and R_{1b} is a hydrogen atom is treated with a halogenating agent,
or, where appropriate, the hydroxyl radical represented by R'₁ₐ is converted by oxidation to an oxo radical, and then, where appropriate, said oxo radical is converted to a hydroxyimino or alkyloxyimino radical, according to known methods, to obtain a quinolylpropylpiperidine derivative of general formula: 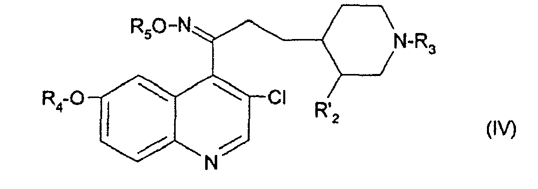
for which R'₂, R₃ and R₄ are as defined above, and R₅ is a hydrogen atom or an alkyl radical, and the derivative of general formula (IV) for which R₅ is a hydrogen atom is reduced to an amine, and, where appropriate, converted to a monoalkylated or dialkylated amine, or, where appropriate, the derivative of general formula (IV) for which R₅ is a hydrogen atom is reduced to a hydroxylamine, or the derivative of general formula (IV) for which R₅ is an alkyl radical to an alkyloxyamine, and then, where appropriate, to obtain the derivative for which R₁ₐ is alkylalkyloxyamino, the derivative obtained for which R₁ₐ is alkyloxyamino is converted by alkylation,
and then, R'₂ is converted to a carboxyl or carboxymethyl radical and/or, where appropriate, the carboxyl radical thus obtained or the protected carboxyl radical which may be represented by R'₂ is reduced to a hydroxymethyl radical and, where appropriate, said hydroxymethyl radical is converted to a carboxyl radical according to the usual methods,
and then, where appropriate, the isomers are separated, where appropriate the acid-protecting radical is removed and/or, where appropriate, the product obtained is converted to a salt.

10. The process as claimed in claim 9, in which the quinolylpropylpiperidine derivative of general formula (II), for which R'₁ₐ is a hydrogen atom, is prepared by oxidation in basic medium of a corresponding derivative for which R'₁ₐ and R'_{1b} are hydrogen atoms, the amine functional group of the piperidine is intermediately protected and R'₂ is as defined above or represents a carboxyl or carboxymethyl radical, and, where appropriate, the carboxyl or carboxymethyl radical is reprotected.

11. The process as claimed in claim 9, in which the quinolylpropylpiperidine derivative of general formula (II) in which R'₁ₐ and R_{1b} form an oxo group is prepared by oxidation according to known methods of a derivative of general formula (II) in which R'₁ₐ represents a hydroxyl radical, obtained as described in claim 10.

12. The process as claimed in claim 9, in which the quinolylpropylpiperidine derivative of general formula (II) for which R'₂ represents a protected carboxymethyl radical, and R'₁ₐ and R_{1b} are hydrogen atoms, is prepared by a process according to which, by the Wittig reaction, a suitable phosphorous ylide is condensed with a piperidine derivative of general formula: 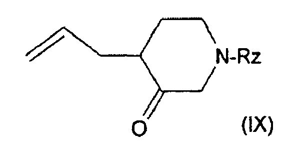
in which Rz represents an amino-protecting radical, to obtain a derivative of formula 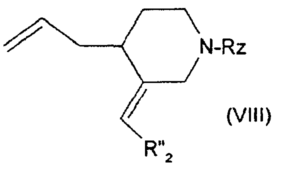
in which Rz is as defined above and R"₂ represents a protected carboxyl radical, which is condensed with a quinoline derivative of general formula: 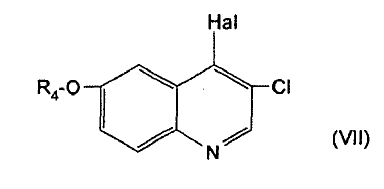
in which R4 is defined as in claim 1 and Hal represents an iodine or bromine atom, to obtain a quinolylpropylpiperidine derivative of general formula: 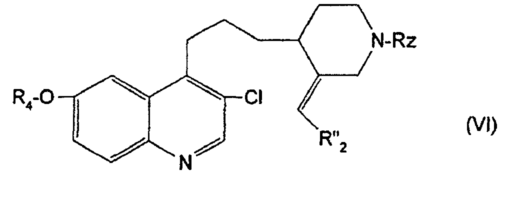
in which R"₂ and Rz are as defined above, which is subjected to a selective hydrogenation and, where appropriate, to an amino-deprotecting reaction.

13. The process as claimed in claim 12, in which the compound of formula (II) is subjected to a reduction of the protected carboxymethyl radical to a hydroxyethyl radical, conversion of said hydroxyethyl radical to a p-toluenesulfonyloxyethyl derivative, and then conversion of this derivative to a vinyl derivative by an elimination reaction followed by oxidation of the derivative obtained and introduction of the protective group on the carboxyl radical thus obtained.

14. The process as claimed in claim 9, in which the quinolylpropylpiperidine derivative of general formula (II), for which R'₁ₐ and R1_{b} are hydrogen atoms, is prepared by allyation of the keto ester of general formula (XIV) 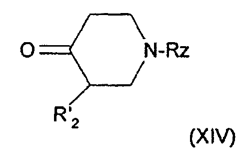
for which R'₂ is as defined in claim 8 and Rz is as defined in claim 12, to obtain a derivative of general formula (XIII): 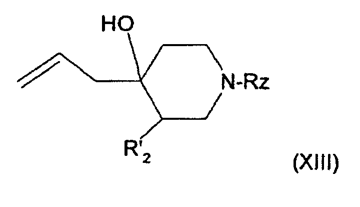
in which R'₂ and Rz are as defined above, which is reacted with an alkyloxalyl halide to obtain a derivative of general formula (XII): 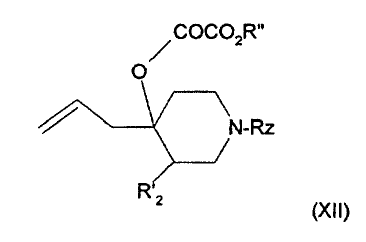
in which R" represents an alkyl radical and R'₂ and Rz are as defined above, which is subjected to a radical deoxygenation reaction, to obtain a derivative of general formula (X): 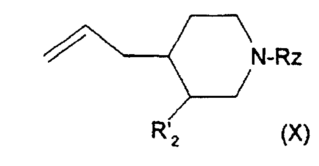
in which R'₂ and Rz are as defined above, which is condensed with a quinoline derivative of general formula (VII) as defined in claim 10, to obtain a derivative of general formula (XI): 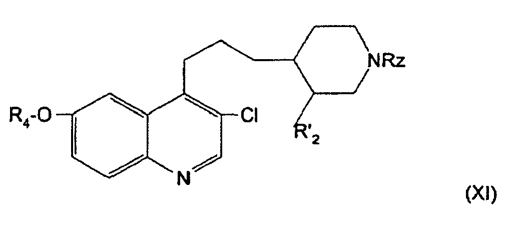
and then the amino-protecting radical Rz is removed.

15. As a medicament, the quinolylpropylpiperidine derivative as defined in claim 1, and also its pharmaceutically acceptable salts.

16. As a medicament, the quinolylpropylpiperidine derivative as defined in any one of claims 2 to 8, and also its pharmaceutically acceptable salts.

17. A pharmaceutical composition containing, as active principle, at least one medicament as claimed in either of claims 15 or 16.

18. A quinolylpropylpiperidine derivative, which corresponds to general formula: 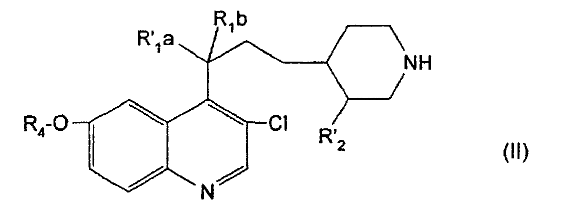
in which R₄ is as defined in claim 1, either R'₁ₐ represents a hydrogen atom or a hydroxyl radical and R_{1b} represents a hydrogen atom or R'₁ₐ and R_{1b} form an oxo group and R'₂ is as defined in claim 9.

19. A quinolylpropylpiperidine derivative, which corresponds to general formula: 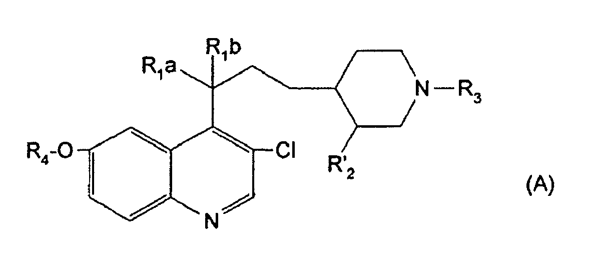
in which R₁ₐ, R_{1b}, R₃ and R₄ are as defined in claim 1 and R'₂ is as defined in claim 9.

20. A quinolylpropylpiperidine derivative, which corresponds to general formula: 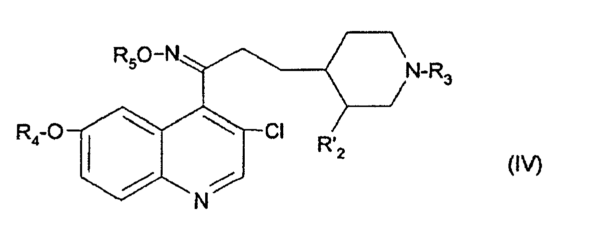
in which R₃ and R₄ are as defined in claim 1 and R'₂ and R₅ are as defined in claim 9.

21. A quinolylpropylpiperidine derivative, which corresponds to general formula: 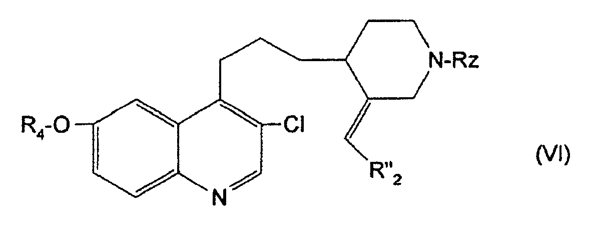
in which R₄ is as defined in claim 1 and R"₂ and Rz are as defined in claim 12.

22. A quinolylpropylpiperidine derivative, which corresponds to general formula: 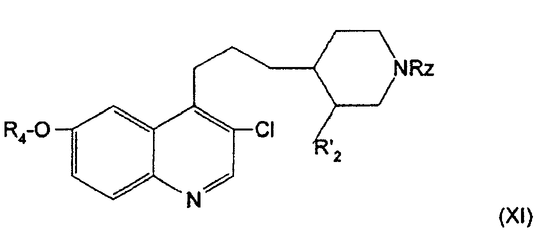
in which R4 is as defined in claim 1, R'₂ is as defined in claim 9 and Rz is as defined in claim 12.

## Patentansprüche

1. Chinolylpropylpiperidin-Derivat, **dadurch gekennzeichnet, dass** es der allgemeinen Formel entspricht 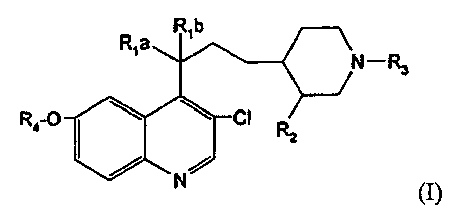
worin:
R₁ₐ ein Wasserstoffatom oder Halogenatom oder einen Hydroxy-, Amino-, Alkylamino-, Dialkylamino-, Hydroxyamino-, Alkyloxyamino- oder Alkylalkyloxyamino-Rest darstellt und R_{1b} ein Wasserstoffatom darstellt, oder R₁ₐ und R_{1b} eine Oxogruppe bilden,
R₂ einen Carboxy-, Carboxymethyl- oder Hydroxymethyl-Rest darstellt,
R₃ einen Alkyl-Rest (1 bis 6 Kohlenstoffatome) darstellt, substituiert mit einem Phenylthio-Rest, der ein bis vier Substituenten tragen kann, ausgewählt aus der Gruppe, bestehend aus Halogen, Hydroxy, Alkyl, Alkyloxy, Trifluormethyl, Trifluormethoxy, Carboxy, Alkyloxycarbonyl, Cyano und Amino, aus einem Cycloalkylthio-Rest, dessen cyclischer Teil 3 bis 7 Kettenglieder aufweist, der einen oder mehrere Substituenten tragen kann, ausgewählt aus Halogen und Trifluormethyl, oder aus einem Heteroarylthio-Rest mit 5 bis 6 Kettengliedern, umfassend 1 bis 4 Heteroatome, ausgewählt aus Stickstoff, Sauerstoff und Schwefel, der einen oder mehrere Substituenten tragen kann, ausgewählt aus der Gruppe, bestehend aus Halogen, Hydroxy, Alkyl, Alkyloxy, Trifluormethyl, Trifluormethoxy, Carboxy, Alkyloxycarbonyl, Cyano und Amino, oder R₃ stellt einen Propargyl-Rest dar, substituiert mit einem Phenyl-Rest, der ein bis vier Substituenten tragen kann, ausgewählt aus der Gruppe, bestehend aus Halogen, Hydroxy, Alkyl, Alkyloxy, Trifluormethyl, Trifluormethoxy, Carboxy, Alkyloxycarbonyl, Cyano und Amino, oder substituiert mit einem Cycloalkyl-Rest, der 3 bis 7 Kettenglieder aufweist, der einen oder mehrere Substituenten tragen kann, ausgewählt aus Halogen und Trifluormethyl, oder substituiert mit einem Heteroaryl-Rest mit 5 bis 6 Kettengliedern, umfassend 1 bis 4 Heteroatome, ausgewählt aus Stickstoff, Sauerstoff und Schwefel, und der einen oder mehrere Substituenten tragen kann, ausgewählt aus der Gruppe, bestehend aus Halogen, Hydroxy, Alkyl, Alkyloxy, Trifluormethyl, Trifluormethoxy, Carboxy, Alkyloxycarbonyl, Cyano und Amino, und
R₄ einen Alkyl-Rest darstellt, bestehend aus 1 bis 6 Kohlenstoffatomen, Alkenyl-CH₂₋oder Alkinyl-CH₂-, deren Alkenyl- oder Alkinyl-Rest 2 bis 6 Kohlenstoffe aufweist, Cycloalkyl oder Cycloalkylalkyl, deren Cycloalkyl-Teil 3 bis 8 Kohlenstoffe aufweist,
in den verschiedenen isomeren, enantiomeren und diastereoisomeren Formen, abgetrennt oder als Mischungen, sowie deren Salze.

2. Derivat der Formel (I), wie definiert nach Anspruch 1, worin R₁ₐ einen Hydroxy-Rest darstellt, und R₂ₐ ein Wasserstoffatom darstellt.

3. Derivat der Formel (I), wie definiert nach Anspruch 1, worin R₁ₐ und R₂ₐ eine Oxogruppe bilden.

4. Derivat der Formel (I), wie definiert nach irgendeinem der Ansprüche 1 bis 3, worin R₄ einen Alkyl-Rest mit 1 bis 6 Kohlenstoffatomen darstellt.

5. Derivat der Formel (I), wie definiert nach irgendeinem der Ansprüche 1 bis 4, worin R₂ einen Carboxy-Rest darstellt.

6. Derivat der Formel (I), wie definiert nach irgendeinem der Ansprüche 1 bis 5, worin R₃ einen Alkyl-Rest, enthaltend 1 bis 6 Kohlenstoffatome, darstellt, substituiert mit einem Phenylthio-, Cycloalkylthio- oder Heteroarylthio-Rest, gegebenenfalls substituiert, wie definiert in Anspruch 1.

7. Derivat der Formel (I), wie definiert nach irgendeinem der Ansprüche 1 bis 6, worin R₃ einen Ethyl-Rest darstellt, substituiert mit einem Thienylthio-Rest, einem halogenierten Phenylthio-Rest, einem Cyclohexylthio-Rest oder einem Cyclopentylthio-Rest.

8. Irgendeines der Derivate der allgemeinen Formel (I), wie definiert nach Anspruch 1, mit den nachfolgenden Namen:
4-[3-Hydroxy-3-(3-chlor-6-methoxychinolin-4-yl)propyl]-1-[2-(2,5-difluorphenylsulfanyl)ethyl]piperidin-3-carbonsäure;
4-[3-Hydroxy-3-(3-chlor-6-methoxychinolin-4-yl)propyl]-1-[2-(2-thienylsulfanyl)-ethyl]piperidin-3-carbonsäure;
in seinen verschiedenen isomeren Formen, abgetrennt oder in Mischungen, genauso wie deren Salze.

9. Verfahren zur Herstellung eines Chinolylpropylpiperidin-Derivats nach Anspruch 1, **dadurch gekennzeichnet, dass** die in Anspruch 1 definierte Kette von R₃ über das Chinolinpropylpiperidin-Derivat der allgemeinen Formel 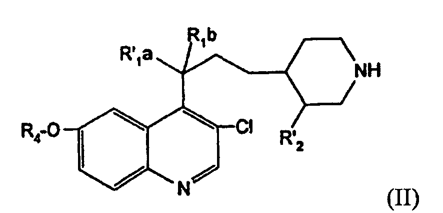
kondensiert wird,
worin R₄ wie in Anspruch 1 definiert ist, wobei R'₁ₐ ein Wasserstoffatom oder einen Hydroxy-Rest darstellt, und R_{1b} ein Wasserstoffatom darstellt, R'₁ₐ und R_{1b} eine Oxogruppe bilden, und R'₂ einen geschützten Carboxy- oder Carboxymethyl-Rest darstellen, um ein Chinolylpropylpiperidin-Derivat der allgemeinen Formel: 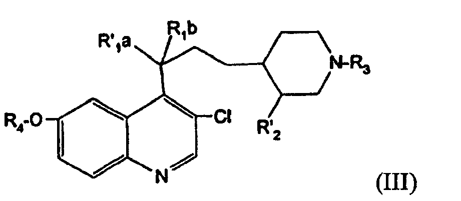
zu erhalten,
worin R'₁ₐ, R_{1b}, R'₂, R₃ und R₄ wie oben definiert sind,
dann gegebenenfalls Behandeln des Derivats, in dem R'₁ₐ einen Hydroxy-Rest darstellt, und R_{1b} ein Wasserstoffatom ist, mit einem Halogenierungsmittel,
oder aber gegebenenfalls Umwandeln des Hydroxy-Restes, dargestellt durch R'₁ₐ, durch Oxidation in einen Sauerstoff-Rest, dann gegebenenfalls Umwandeln dieses in einen Hydroxyimino- oder Alkyloxyimino-Rest gemäß den bekannten Verfahren, um ein Chinolylpropylpiperidin-Derivat der allgemeinen Formel: 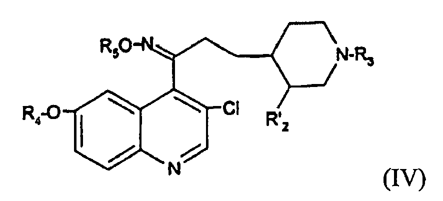
zu erhalten,
worin R'₂, R₃ und R₄ wie zuvor definiert sind, und R₅ ein Wasserstoffatom oder einen Alkyl-Rest darstellt, und Reduzieren des Derivats der allgemeinen Formel (IV), worin R₅ ein Wasserstoffatom darstellt, zu einem Amin, sowie gegebenenfalls Umwandeln in ein monoalkyliertes oder dialkyliertes Amin, oder gegebenenfalls Reduzieren des Derivats der allgemeinen Formel (IV), worin R₅ ein Wasserstoffatom darstellt, in ein Hydroxylamin, oder des Derivats der allgemeinen Formel (IV), worin R₅ einen Alkyl-Rest darstellt, in ein Alkyloxyamin, dann gegebenenfalls Umwandeln des erhaltenen Derivats, worin R₁ₐ ein Alkyloxyamin darstellt, durch Alkylierung, um das Derivat zu erhalten, worin R₁ₐ Alkylalkoxyamin darstellt,
dann Umwandeln von R'₂ in einen Carobxy- oder Carboxymethyl-Rest, und/oder gegebenenfalls Reduzieren des so erhaltenen Carboxy-Rests oder des geschützten Carboxy-Rests, den R'₂ darstellen kann, in einen Hydroxymethyl-Rest, und gegebenenfalls Umwandeln dieses in einen Carboxymethyl-Rest nach den gängigen Verfahren,
dann gegebenenfalls Abtrennen der Isomeren, gegebenenfalls Eliminieren des Schutzrestes der Säure und/oder gegebenenfalls Umwandeln des erhaltenen Produkts in ein Salz.

10. Verfahren nach Anspruch 9, **dadurch gekennzeichnet, dass** das Chinolylpropylpiperidin-Derivat der allgemeinen Formel (II), worin R'₁ₐ ein Wasserstoffatom darstellt, hergestellt wird durch Oxidation in basischem Milieu aus einem entsprechenden Derivat, worin R'₁ₐ und R_{1b} Wasserstoffatome darstellen, die Amin-Funktion des Piperidins vorübergehend geschützt wird, und R'₂ so wie zuvor definiert ist, oder einen Carboxy- oder Carboxymethyl-Rest darstellt, und gegebenenfalls Entschützen des Carboxy- oder Carboxymethyl-Rests.

11. Verfahren nach Anspruch 9, **dadurch gekennzeichnet, dass** das Chinolylpropylpiperidin-Derivat der allgemeinen Formel (II), worin R'₁ₐ und R_{1b} eine Oxogruppe darstellen, hergestellt wird durch Oxidation gemäß den bekannten Verfahren aus einem Derivat der allgemeinen Formel (II), worin R'₁ₐ einen Hydroxy-Rest darstellt, erhalten wie in Anspruch 10 beschrieben.

12. Verfahren nach Anspruch 9, **dadurch gekennzeichnet, dass** das Chinolylpropylpiperidin-Derivat der allgemeinen Formel (II), worin R'₂ einen geschützten Carboxymethyl-Rest darstellt, und R'₁ₐ und R_{1b} Wasserstoffatome darstellen, hergestellt wird durch ein Verfahren, nach dem durch Witting-Reaktion ein geeignetes Phosphorylid über ein Piperidin-Derivat der allgemeinen Formel: 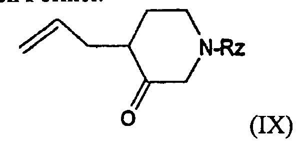
kondensiert wird, worin Rz einen Schutzrest für das Amin darstellt, um ein Derivat der Formel 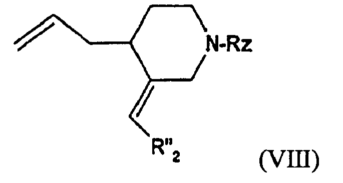
zu erhalten, worin Rz wie oben definiert ist und R"₂ einen geschützten Carboxy-Rest darstellt, das zu einem Chinolin-Derivat der allgemeinen Formel: 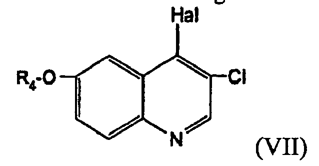
kondensiert wird, worin R₄ wie in Anspruch 1 definiert ist, und Hal ein Iod- oder Bromatom darstellt, um ein Chinolylpropylpiperidin-Derivat der allgemeinen Formel: 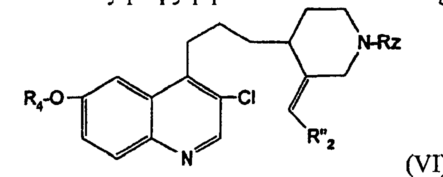
zu erhalten, worin R"₂ und Rz wie zuvor definiert sind, das einer selektiven Hydrierung und gegebenenfalls einer Entschützungsreaktion am Amin unterzogen wird.

13. Verfahren nach Anspruch 12, **dadurch gekennzeichnet, dass** die Verbindung der Formel (II) einer Reduktion des geschützten Carboxymethyl-Rests zu einem Hydroxyethyl-Rest unterzogen wird, Umwandeln dieses in ein p-Toluolsulfonyloxyethyl-Derivat, dann Umwandeln dieses Derivats in ein Vinyl-Derivat durch Eliminationsreaktion, gefolgt von der Oxidation des erhaltenen Derivats, und dann Einführung einer Schutzgruppe für den so erhaltenen Carboxyl-Rest.

14. Verfahren nach Anspruch 9, **dadurch** gekennezeichnet, dass das Chinolylpropylpiperidin-Derivat der allgemeinen Formel (II), worin R'₁ₐ und R_{1b} Wasserstoffatome darstellen, hergestellt wird durch Allylierung des Ketoesters der allgemeinen Formel (XIV) 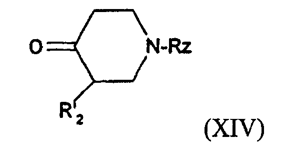
worin R'₂ wie in Anspruch 8 definiert ist, und Rz wie in Anspruch 12 definiert ist, um ein Derivat der allgemeinen Formel (XIII): 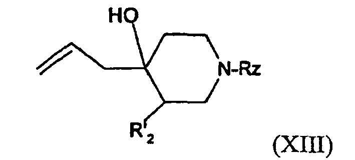
zu erhalten,
worin R'₂ und Rz wie zuvor definiert sind, wobei durch Einwirken eines Alkyloxalyl-Halogenids ein Derivat der allgemeinen Formel (XII): 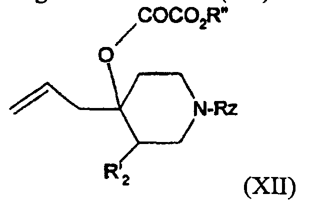
erhalten wird,
worin R" einen Alkyl-Rest darstellt, und R'₂ und Rz wie zuvor definiert sind, das einer radikalischen Desoxidierungsreaktion unterzogen wird, um ein Derivat der allgemeinen Formel (X): 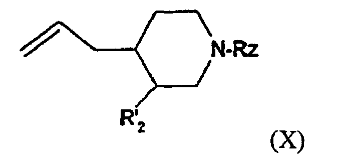
zu erhalten,
worin R'₂ und Rz wie zuvor definiert sind, das mit einem Chinolin-Derivat der allgemeinen Formel (VII), definiert wie in Anspruch 10, kondensiert wird, um ein Derivat der allgemeinen Formel (XI): 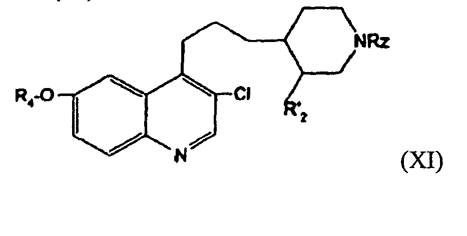
zu erhalten, dann Eliminieren des Rz-Schutzrests des Amins.

15. Chinolylpropylpiperidin-Derivate, wie definiert nach Anspruch 1, als Arzneimittel, ebenso wie deren pharmazeutisch verträgliche Salze.

16. Chinolylpropylpiperidin-Derivate, wie definiert nach irgendeinem der Ansprüche 2 bis 8, als Arzneimittel, ebenso wie deren pharmazeutisch verträgliche Salze.

17. Pharmazeutische Zusammensetzungen, enthaltend mindestens ein Arzneimittel nach einem der Ansprüche 15 und 16 als Wirkstoff.

18. Chinolylpropylpiperidin-Derivat, **dadurch gekennzeichnet, dass** es der allgemeinen Formel entspricht: 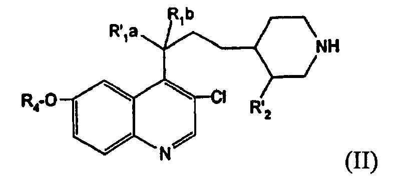
worin R₄ wie in Anspruch 1 definiert ist, R'₁ₐ ein Wasserstoffatom oder einen Hydroxy-Rest darstellt, und R_{1b} ein Wasserstoffatom darstellt, R'₁ₐ und R_{1b} eine Oxogruppe bilden, und R'₂ wie in Anspruch 9 definiert ist.

19. Chinolylpropylpiperidin-Derivat, **dadurch gekennzeichnet, dass** es der allgemeinen Formel entspricht: 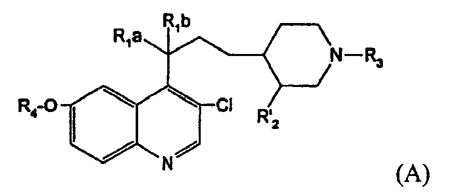
worin R₁ₐ, R_{1b}, R₃ und R₄ wie in Anspruch 1 definiert sind, und R'₂ wie in Anspruch 9 definiert ist.

20. Chinolylpropylpiperidin-Derivat, **dadurch gekennzeichnet, dass** es der allgemeinen Formel entspricht: 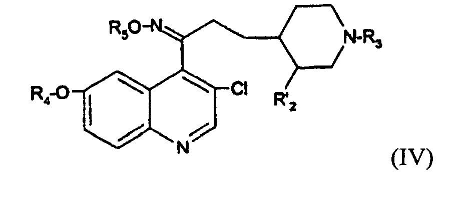
worin R₃ und R₄ wie in Anspruch 1 definiert sind, und R'₂ und R₅ wie in Anspruch 9 definiert sind.

21. Chinolylpropylpiperidin-Derivat, **dadurch gekennzeichnet, dass** es der allgemeinen Formel entspricht: 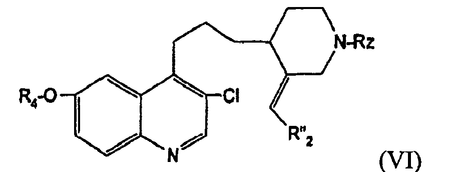
worin R₄ wie in Anspruch 1 definiert ist, und R"₂ und Rz wie in Anspruch 12 definiert sind.

22. Chinolylpropylpiperidin-Derivat, **dadurch gekennzeichnet, dass** es der allgemeinen Formel entspricht: 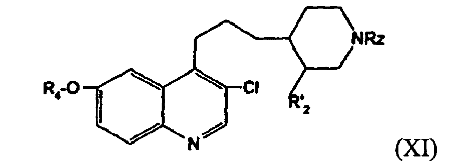
worin R₄ wie in Anspruch 1 definiert ist, R'₂ wie in Anspruch 9 definiert ist und Rz wie in Anspruch 12 definiert ist.
